(19) Europäisches Patentamt
European Patent Office
Office européen des brevets

(11) **EP 2 488 589 B2**

(12) # NEUE EUROPÄISCHE PATENTSCHRIFT
Nach dem Einspruchsverfahren

(45) Veröffentlichungstag und Bekanntmachung des Hinweises auf die Entscheidung über den Einspruch:
**10.04.2024   Patentblatt 2024/15**

(45) Hinweis auf die Patenterteilung:
**21.12.2016   Patentblatt 2016/51**

(21) Anmeldenummer: **10765585.4**

(22) Anmeldetag: **12.10.2010**

(51) Internationale Patentklassifikation (IPC):
**C09C 1/00** (2006.01)    **A61K 8/29** (2006.01)
**A61Q 1/02** (2006.01)    **A61Q 1/10** (2006.01)
**A61Q 3/02** (2006.01)    **A61Q 19/00** (2006.01)

(52) Gemeinsame Patentklassifikation (CPC):
**C09C 1/0021; A61K 8/29; A61Q 19/00;**
A61K 2800/436; A61Q 1/02; A61Q 1/10; A61Q 3/02;
C01P 2006/62; C01P 2006/80; C09C 2200/1004;
C09C 2200/102; C09C 2200/301; C09C 2200/302;
C09C 2220/106

(86) Internationale Anmeldenummer:
**PCT/EP2010/006237**

(87) Internationale Veröffentlichungsnummer:
**WO 2011/045030 (21.04.2011 Gazette 2011/16)**

(54) **PERLGLANZPIGMENTE AUF BASIS VON FEINEN UND DÜNNEN SYNTHETISCHEN SUBSTRATEN**

PEARLESCENT PIGMENT BASED ON FINE, THIN SUBSTRATES

PIGMENTS NACRÉS À BASE DE SUBSTRATS FINS ET MINCES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.10.2009   DE 102009049413**

(43) Veröffentlichungstag der Anmeldung:
**22.08.2012   Patentblatt 2012/34**

(73) Patentinhaber: **Eckart GmbH**
**91235 Hartenstein (DE)**

(72) Erfinder:
• **SCHMIDT, Ulrich**
**91217 Hersbruck (DE)**
• **GRÜNER, Michael**
**91275 Auerbach (DE)**
• **SCHUMACHER, Dirk**
**91257 Pegnitz (DE)**
• **KAUPP, Günter**
**91284 Neuhaus (DE)**

• **MENDLER, Barbara**
**88709 Meersburg (DE)**

(74) Vertreter: **Strych, Sebastian et al**
**Mitscherlich PartmbB**
**Patent- und Rechtsanwälte**
**Karlstraße 7**
**80333 München (DE)**

(56) Entgegenhaltungen:
**EP-A- 1 564 261        WO-A-02/090448**
**WO-A-2007/054379    WO-A1-2009/127406**

• **NANCY M. HEPP , WILLIAM R. MINDAK , JOHN CHENG: "Determination of total lead in lipstick: Development and validation of a microwave-assisted digestion, inductively coupled plasma-mass spectrometric method", JOURNAL OF THE SOCIETY OF COSMETIC CHEMISTS, vol. 60, no. 4, July 2009 (2009-07), - August 2009 (2009-08), pages 405-414,**

EP 2 488 589 B2

**Beschreibung**

Die Erfindung betrifft Perlglanzpigmente basierend auf weitgehend transparenten plättchenförmigen synthetischen Substraten, deren Verwendung und Herstellung sowie Beschichtungsmittel, die die erfindungsgemäßen Perlglanzpigmente enthalten.

Die EP 0 723 997 B1 beschreibt ein perlenartig glänzendes flockenartiges Pigment, welches einen synthetisch hergestellten Fluorophlogopit-Glimmer, der mit Metalloxid beschichtet ist, umfasst. Der synthetisch hergestellte Fluorophlogopit-Glimmer weist einen mittleren Brechungsindex, der den Wert 1,58 nicht übersteigt, einen Eisengehalt mit einem Gewichtsanteil von höchstens 0,1 Gew.-% sowie ein Perlparameter = Spezifisches Volumen (A) x Glanzwert des Pulvers (B) ≥ 10 auf. Derartige Pigmente sollen einen reduzierten Gelbstich und einen verbesserten Interferenzeffekt aufweisen.

Interferenzpigmente auf der Basis von transparenten plättchenförmigen Substraten, die mit einer hochbrechenden Beschichtung bestehend aus Titandioxid belegt sind werden in EP 1 564 261 A2 beschrieben. Die plättchenförmigen Substrate weisen eine mittlere Dicke zwischen 0,02 $\mu$m und 2 $\mu$m, vorzugsweise zwischen 0,1 $\mu$m und 1 $\mu$m und besonders bevorzugt zwischen 0,2 $\mu$m und 0,8 $\mu$m auf. Zur Erzielung eines intensiven Farbeffekts mit überlagerter, winkelabhängiger Farbtönung muss die mittlere Dicke der einzelnen Plättchen innerhalb einer Standardabweichung von ≤ 15 % liegen.

In der EP 1 072 651 A1 werden Pigmente auf Basis dünner Flocken mit einer mittleren Partikelgröße von 0,5 $\mu$m bis 10 $\mu$m, vorzugsweise 2 $\mu$m bis 8 $\mu$m beschrieben, die zunächst mit kugelförmigen $SiO_2$-Partikeln und nachfolgend mit ultrafeinen Titandioxidpartikeln beschichtet sind. Derartige Pigmente werden u.a. aufgrund ihres Softfokuseffekts als Füllstoffe beispielsweise kosmetischen Formulierungen zugesetzt. Durch die kugelförmige Struktur der $SiO_2$- und $TiO_2$-Partikel erfolgt im Wesentlichen eine ungerichtete Reflexion, die in der kosmetischen Applikation auf der Haut einen unerwünschten whitening-Effekt hervorruft.

Die vorgenannten Perlglanzpigmente werden unter anderem auch zur Pigmentierung von Kosmetika verwendet. Nachteiligerweise weisen diese bekannten Perlglanzpigmente keinen ausreichenden Softfokuseffekt auf, d.h. vermögen es in einem Kosmetikum nicht, Falten oder Unregelmäßigkeiten der Haut zufriedenstellend abzudecken, ohne gleichzeitig einen unerwünschten whitening-Effekt hervorzurufen. Des Weiteren weisen marktübliche Perlglanzpigmente einen messbaren Bleigehalt auf, der in kosmetischen Formulierungen unerwünscht ist.

Verunreinigungen durch Schwermetalle in kosmetischen Formulierungen sind im Interesse des Verbrauchers unerwünscht. Insbesondere erhöhte Bleigehalte in kosmetischen Formulierungen sind in jüngster Zeit in Kritik geraten. Farbadditive werden bzgl. ihres Bleigehalts von der FDA überwacht und dürfen einen Grenzwert von 20 $\mu$g/ g nicht überschreiten. Weitere kosmetische Inhaltsstoffe unterliegen im Hinblick auf den Bleigehalt der Verantwortung der Hersteller (Nancy M. Hepp, William R. Mindak, John Cheng, J. Cosmet. Sci., 60, 405 - 414 (July/ August 2009)).

Die WO 2007/054379 A1 betrifft Interferenzpigmente auf der Basis von plättchenförmigen Glassubstraten mit einer durchschnittlichen Dicke von < 1 $\mu$m.

Die WO 02/090448 A2 betrifft Effektpigmente auf der Basis von Glasplättchen mit einer Dicke von ≤ 1,0 $\mu$m.

Die EP 1 564 261 A2 betrifft Interferenzpigmente auf der Basis von niedrigbrechenden transparenten plättchenförmigen Substraten, die eine hochbrechende Beschichtung bestehend aus $TiO_2$ mit einer Schichtdicke von 20 - 200 nm aufweisen.

Die WO 2009/127406 A1 stellt einen Stand der Technik gemäß Art. 54(3) EPÜ dar und betrifft Perlglanzpigmente auf Basis von feinen und dünnen Substraten.

Hepp N. M. et al. (J. Cosmet. Sci., 60, 405-414 (Juli/August 2009)) betrifft die Bestimmung des Bleigehaltes in Lippenstift.

Es besteht mithin ein Bedarf an verbesserten Perlglanzpigmenten mit einem möglichst geringen Gesamtbleigehalt. Insbesondere ist es erwünscht, Perlglanzpigmente mit verbessertem Softfokus und angenehmem Hautgefühl bereitzustellen. Diese Perlglanzpigmente sollen die Eigenschaften von herkömmlichen Perlglanzpigmenten wie z.B. Interferenz, Tiefenglanz, gegebenenfalls Farbigkeit, mit zusätzlichem Softfokuseffekt vereinen.

Schließlich sollten die Perlglanzpigmente eine gute Deckfähigkeit bei starkem Haze-Effekt sowie gleichzeitig intensiver Interferenzfarbe aufweisen.

Die der Erfindung zugrundeliegende Aufgabe wird durch die Bereitstellung von Perlglanzpigmenten umfassend ein weitgehend transparentes plättchenförmiges synthetisches Substrat mit einer Dichte $\rho_S$ und mindestens einer optisch wirksamen Beschichtung mit einer Dichte $\rho_M$ gelöst, wobei das Substrat eine mittlere Größe $d_{50}$ aus einem Bereich von 2,0 $\mu$m bis 8,0 $\mu$m, eine mittlere Höhe $h_s$ aus einem Bereich von 40 nm bis 110 nm aufweist und der Gesamtbleigehalt der Perlglanzpigmente bei ≤ 10 ppm liegt, wobei die optisch wirksame Beschichtung eine hochbrechende Beschichtung mit einem Brechungsindex $n_M$ > 2,0 ist und die optisch wirksame Schicht eine Metalloxidschicht aufweist oder ist, wobei a) die Metalloxidschicht aus $TiO_2$ und das Substrat aus synthetischem Glimmer besteht und folgender Zusammenhang zwischen dem $TiO_2$-Gehalt in Gew.-%, bezogen auf das Gesamtgewicht von $TiO_2$ und synthetischem Glimmer, und der mittleren Schichtdicke der $TiO_2$-Beschichtung besteht:

ein Metalloxidgehalt von 30 - 80 Gew.-% bei einer mittleren Metalloxidschichtdicke von über 20 bis 50 nm;

ein Metalloxidgehalt von 50 - 85 Gew.-% bei einer mittleren Metalloxidschichtdicke von über 50 bis 75 nm;

ein Metalloxidgehalt von 59 - 89 Gew.-% bei einer mittleren Metalloxidschichtdicke von über 75 bis 95 nm;

ein Metalloxidgehalt von 66 - 92 Gew.-% bei einer mittleren Metalloxidschichtdicke von über 95 bis 125 nm;

ein Metalloxidgehalt von 69 - 96 Gew.-% bei einer mittleren Metalloxidschichtdicke von über 125 bis 215 nm oder

wobei b) die Metalloxidschicht aus $Fe_2O_3$ und das Substrat aus synthetischem Glimmer besteht und folgender Zusammenhang zwischen dem $Fe_2O_3$-Gehalt in Gew.-%, bezogen auf das Gesamtgewicht von $Fe_2O_3$ und synthetischem Glimmer, und der mittleren Schichtdicke der $Fe_2O_3$-Beschichtung:

ein $Fe_2O_3$-Gehalt von 47,5 - 72,4 Gew.-% bei einer mittleren $Fe_2O_3$-Schichtdicke von über 35 bis 45 nm;

ein $Fe_2O_3$-Gehalt von 57,5 - 82,4 Gew.-% bei einer mittleren $Fe_2O_3$-Schichtdicke von über 45 bis 55 nm;

ein $Fe_2O_3$-Gehalt von 62,5 - 87,4 Gew.-% bei einer mittleren $Fe_2O_3$-Schichtdicke von über 55 bis 65 nm.

Bevorzugte Weiterbildungen der erfindungsgemäßen Perlglanzpigmente sind in den Unteransprüchen 2 bis 3 angegeben.

Die der Erfindung zugrundeliegende Aufgabe wird auch durch ein Verfahren zur Herstellung der erfindungsgemäßen Perlglanzpigmente gelöst, das folgende Schritte umfasst:

a) Klassieren des weitgehend transparenten plättchenförmigen synthetischen Substrates unter Erhalt eines Substrates mit einer mittleren Höhe $h_s$ aus einem Bereich von 40 nm bis 110 nm,
b) Beschichten des klassierten weitgehend transparenten plättchenförmigen synthetischen Substrates mit mindestens einer optisch wirksamen, vorzugsweise hochbrechenden, Schicht unter Erhalt eines Perlglanzpigmentes mit einer mittleren Größe $d_{50}$ aus einem Bereich von 2,1 $\mu$m bis 8,6 $\mu$m.

Weiterhin wird die Aufgabe der Erfindung auch durch die Verwendung der erfindungsgemäßen Perlglanzpigmente in Lacken, Druckfarben, Tintenstrahltinten, Tonern, Kosmetika, Kunststoffen, Textilien, Glas, Email, Glasuren oder Keramik gelöst.

Auch wird die der Erfindung zugrundeliegende Aufgabe durch Bereitstellung eines Beschichtungsmittels, insbesondere eines kosmetischen Präparats gelöst, das die erfindungsgemäßen Perlglanzpigmente enthält.

Die Begriffe "Beschichtung" und "Schicht" werden bei der vorliegenden Erfindung austauschbar verwendet.

Die Erfinder haben überraschend festgestellt, dass Perlglanzpigmente auf Basis von weitgehend transparenten plättchenförmigen synthetischen Substraten einen guten Softfokuseffekt und ein gutes Hautgefühl aufweisen, wenn der $d_{50}$-Wert der Größe und die mittlere Höhe $h_s$ jeweils innerhalb des vorstehend angegebenen Wertebereichs liegen.

Die erfindungsgemäßen Perlglanzpigmente weisen mithin ein Substrat mit einem außerordentlich geringen Durchmesser und einer außerordentlich geringen Substratdicke auf. Es hat sich überraschend gezeigt, dass bei Verwendung dieser Substrate zur Herstellung von Perlglanzpigmenten, Perlglanzpigmente mit einem guten Softfokuseffekt und einem angenehmen Hautgefühl erhalten werden.

Zugleich weisen die erfindungsgemäßen Perlglanzpigmente ungeachtet des geringen Durchmessers starke Interferenzfarben auf, so dass die erfindungsgemäßen Perlglanzpigmente insbesondere zur Farbgebung von Kosmetika geeignet sind.

Kosmetika, welche die erfindungsgemäßen Perlglanzpigmente enthalten zeichnen sich beispielsweise in Make-up durch die Verleihung eines homogenen Aussehens aus. Aufgrund der Transparenz der erfindungsgemäßen Perlglanzpigmente wird der Haut ein natürliches Aussehen ohne Masking-Effekt verliehen. Die geringe Teilchengröße der erfindungsgemäßen Perlglanzpigmente verhindert einen unerwünschten Glanz bei gleichzeitiger Deckkraft und gegebenenfalls Farbigkeit.

Ferner hat sich überraschend herausgestellt, dass die erfindungsgemäßen Perlglanzpigmente bei der Verwendung in Kosmetika zuverlässiger an dem Untergrund, beispielsweise Haut, Augenlidern, Haaren, Wimpern, Finger- und/oder Fußnägeln, haften, obgleich die Kontaktfläche zum Untergrund aufgrund des geringeren Perlglanzpigmentdurchmessers geringer ist.

Im Hinblick auf dieses überraschende Ergebnis bei der Haftung am Untergrund wird nunmehr vermutet, dass bei größeren Perlglanzpigmenten diese nicht vollflächig an dem Untergrund anliegen und mithin Abschnitte dieser größeren Perlglanzpigmente über den Untergrund "überstehen" und bei Einwirkung von mechanischen Kräften, beispielsweise bei einem Lidschlag oder Muskelbewegungen, oder unter dem Einfluss von Flüssigkeiten, beispielsweise Wasser oder Körpersekreten, wie zum Beispiel Schweiß, größere Perlglanzpigmente leichter vom Untergrund abgelöst werden. Insbesondere eine reifere bzw. ältere Haut weist stärkere Unregelmäßigkeiten auf, an die sich die erfindungsgemäßen Perlglanzpigmente vermutlich vollflächig oder mit einem größeren relativen Flächenanteil anhaften können, als es größere Perlglanzpigmente vermutlich vermögen.

Bei Haaren oder Wimpern, die üblicherweise eine Dicke im Bereich von 40 μm bis 120 μm aufweisen, liegen die erfindungsgemäßen Perlglanzpigmente auch unter Berücksichtigung der Krümmung der Haare, vermutlich weitgehend vollflächig an den Haaren bzw. Wimpern an. Die erfindungsgemäßen Perlglanzpigmente haben somit überraschenderweise einen sehr guten Halt an Haaren und Wimpern, so dass diese nicht beim Kämmen oder beim Verwirbeln von Haaren, beispielsweise im Wind, abfallen. Ein Abfallen der Perlglanzpigmente wäre äußerst nachteilig, da zum einen die Haare oder Wimpern nicht mehr die gewünschte einheitliche Farbgebung aufweisen und zum anderen der optische Eindruck von Haarschuppen entsteht, beispielsweise durch Ablagerung der abgefallenen Perlglanzpigmente im Schulterbereich eines Kleides oder Anzugs.

Unter einer optisch wirksamen Beschichtung werden erfindungsgemäß beispielsweise semitransparente Metallschichten verstanden. Die Schichtdicke der semitransparenten Metallschichten liegt üblicherweise in einem Bereich von 5 nm bis 30 nm, vorzugsweise in einem Bereich von 10 nm bis 25 nm. Auch haben sich Schichtdicken aus einem Bereich von 20 nm bis 25 nm als sehr geeignet erwiesen.

Ferner werden unter einer optisch wirksamen Beschichtung erfindungsgemäß hochbrechende Metalloxidschichten verstanden. Der Brechungsindex von hochbrechenden Metalloxidschichten liegt oberhalb von 2,0. Als sehr geeignet haben sich auch Brechungsindices von mehr als 2,2 oder mehr als 2,6 erwiesen. Vorzugsweise liegt die geometrische Schichtdicke der hochbrechenden Metalloxidschicht in einem Bereich zwischen 10 nm und 300 nm, weiter bevorzugt in einem Bereich zwischen 20 nm und 200 nm, noch weiter bevorzugt in einem Bereich zwischen 50 nm und 150 nm. Anstelle hochbrechender Metalloxide können gemäß der vorliegenden Offenbarung auch andere hochbrechende Materialien, beispielsweise Metallsulfide, Metallselenide oder Metallnitride verwendet werden, wobei die geometrischen Schichtdicken vorzugsweise die für die hochbrechenden Metalloxide angegebenen Bereiche aufweisen.

Erfindungsgemäß ist die optisch wirksame Beschichtung eine hochbrechende Metalloxidschich mit einem Brechungsindex $n_M > 2,0$

Selbstverständlich kann auch mehr als eine hochbrechende Metalloxidschicht auf dem Substrat aufgebracht sein. Vorzugsweise wird bei dieser Variante zwischen zwei hochbrechenden Metalloxidschichten wenigstens eine niedrigbrechende Schicht angeordnet, die vorzugsweise einen Brechungsindex von kleiner als 1,8, weiter bevorzugt von kleiner als 1,6 aufweist.

Als niedrigbrechende Schicht werden vorzugsweise niedrigbrechende Metalloxidschichten, insbesondere Siliciumoxid und/oder Aluminiumoxid, verwendet.

Als niedrigbrechende Schichten werden vorzugsweise Schichten aus Siliciumoxid, vorzugsweise $SiO_2$, Aluminiumoxid, vorzugsweise $Al_2O_3$, AlOOH, Boroxid, vorzugsweise $B_2O_3$, $MgF_2$ oder Mischungen davon verwendet.

Das Substrat weist eine mittlere Größe $d_{50}$ aus einem Bereich von 2,0 μm bis 8,0 μm, bevorzugt aus einem Bereich von 2,1 μm bis 7,0 μm, weiter bevorzugt aus einem Bereich von 2,5 μm bis 6,0 μm und besonders bevorzugt aus einem Bereich von 3,0 μm bis 5,0 μm auf.

Oberhalb einer mittleren Größe $d_{50}$ von 8,0 μm sind die vorteilhaften Eigenschaften der erfindungsgemäßen Perlglanzpigmente, wie beispielsweise die Anhaftung an runden Oberflächen wie Wimpern, nicht mehr erkennbar.

Unterhalb einer mittleren Größe $d_{50}$ von 2,0 μm sind die vorteilhaften Eigenschaften der erfindungsgemäßen Perlglanzpigmente nicht mehr erkennbar, da durch die steigende Teilchenanzahl pro Gramm ein zunehmender Anteil an ungerichteter Lichtstreuung und somit ein unerwünschter whitening-Effekt resultiert.

Unter der mittleren Größe $d_{50}$ wird im Rahmen dieser Erfindung der $d_{50}$-Wert der Summenhäufigkeitsverteilung der volumengemittelten Größenverteilungsfunktion, wie sie durch Laserbeugungsmethoden erhalten werden, verstanden. Bevorzugt wird hierbei die Größenverteilungskurve mit einem Gerät der Firma Quantachrome (Gerät: Cilas 1064) bestimmt. Die Auswertung der Streulichtsignale erfolgte dabei über die Fraunhofer-Methode.

Der $d_{50}$-Wert gibt an, dass 50 % der Substrat- bzw. Pigmentpartikel einen Durchmesser aufweisen, der gleich oder kleiner als der angegebene Wert, beispielsweise 6 μm, ist.

Weiterhin weist das Substrat der erfindungsgemäßen Perlglanzpigmente eine mittlere Höhe (Schichtdicke) $h_s$ aus einem Bereich von 40 nm bis 110 nm, bevorzugt aus einem Bereich von 40 nm bis 100 nm, weiter bevorzugt aus einem Bereich von 40 nm bis 95 nm, noch weiter bevorzugt von aus einem Bereich von 45 nm bis 94 nm und besonders bevorzugt aus einem Bereich von 50 nm bis 90 nm auf.

Unterhalb von 40 nm Schichtdicke sind die Perlglanzpigmente mechanisch zu zerbrechlich und die Beschichtungszeiten mit Metall oder hochbrechendem Metalloxid dauern aufgrund der extrem hohen spezifischen Oberfläche zu lange, um wirtschaftlich vertretbar zu sein. Unter der spezifischen Oberfläche wird die Oberfläche pro Gewichtseinheit verstanden. Da die Schichtdicke der Substrate der erfindungsgemäßen Perlglanzpigmente äußerst gering ist, weisen diese Substrate pro Gewichtseinheit eine sehr große Oberfläche im Vergleich zu herkömmlichen Substraten auf.

Ab einer Schichtdicke von 110 nm sind die Vorteile der Erfindung kaum noch vorhanden.

In einer weiteren Ausführungsform weist das erfindungsgemäße Perlglanzpigment eine Standardabweichung der Substrathöhe $h_s$ von 25 % bis 80 %, bevorzugt von 30 % bis 60% und besonders bevorzugt von 28 % bis 50 % auf.

Gemäß einer bevorzugten Variante der Erfindung weisen die Perlglanzpigmente eine Größenverteilung mit einem $d_{90}$-Wert aus einem Bereich von 5,0 μm bis 11,0 μm, bevorzugt aus einem Bereich von 5,1 μm bis 10,0 μm, weiter bevorzugt aus einem Bereich von 5,5 μm bis 9,0 μm und besonders bevorzugt aus einem Bereich von 6,0 μm bis 8,5 μm auf.

Gemäß einer weiteren Variante der Erfindung weisen die Perlglanzpigmente eine Größenverteilung mit einem $d_{50}$-Wert aus einem Bereich von 2,1 μm bis 8,6 μm, bevorzugt aus einem Bereich von 2,2 μm bis 7,6 μm, weiter bevorzugt aus einem Bereich von 2,6 μm bis 6,6 μm und besonders bevorzugt aus einem Bereich von 3,1 μm bis 5,6 μm auf.

Bei den erfindungsgemäßen Perlglanzpigmenten handelt es sich mithin um eine neue Klasse an Perlglanzpigmenten in Form von äußerst feinen Perlglanzpigmenten, die auf einem weitgehend transparenten Substrat mit einer sehr geringen mittleren Größe und einer sehr geringen mittleren Schichtdicke basieren. Derartige Pigmente weisen aufgrund des hohen Anteils an Kanten, relativ zur Fläche, einen ungewöhnlich hohen Streulichtanteil auf. Dies führt beispielsweise in Lackapplikationen zu einem hohen Haze-Effekt. Überraschenderweise weisen die erfindungsgemäßen Perlglanzpigmente dennoch eine intensive Interferenzfarbe auf, obwohl die Interferenzfarbe durch Streueffekte üblicherweise verhindert bzw. stark abgeschwächt wird.

Die erfindungsgemäßen Perlglanzpigmente weisen mindestens eine optisch wirksame Beschichtung, vorzugsweise in Form einer hochbrechenden Beschichtung, bevorzugt hochbrechenden Metalloxidschicht, und/oder einer semitransparenten Metallbeschichtung mit einer Dichte $\rho_M$ auf. Unter der Dichte $\rho_M$ wird die Dichte der optisch wirksamen Beschichtung verstanden. So steht $\rho_M$ im Falle einer Metalloxidschicht für die Dichte der Metalloxidschicht und im Falle einer semitransparenten Metallschicht für die Dichte der semitransparenten Metallschicht.

Im Rahmen dieser Erfindung werden auch Pigmente basierend auf weitgehend transparenten plättchenförmigen synthetischen Substraten und einer semitransparenten Metallschicht als Perlglanzpigmente bezeichnet. Vorzugsweise weisen die erfindungsgemäßen Perlglanzpigmente einen Interferenzeffekt auf.

Geeignete zu beschichtende weitgehend transparente plättchenförmige Substrate sind nichtmetallische, synthetische plättchenförmige Substrate. Die Substrate sind vorzugsweise im Wesentlichen transparent, d.h. für sichtbares Licht sind sie zumindest teilweise durchlässig.

Gemäß einer bevorzugten Ausführungsform der Erfindung können die weitgehend transparenten plättchenförmigen synthetischen Substrate aus der Gruppe, bestehend aus synthetischem Glimmer, $SiO_2$-Plättchen, $Al_2O_3$-Plättchen, Polymerplättchen, plättchenförmigem Bismuthoxychlorid, plättchenförmigen Substraten umfassend eine anorganisch-organische Mischschicht, und deren Gemische, ausgewählt werden. Bevorzugt werden die weitgehend transparenten plättchenförmigen synthetischen Substrate aus der Gruppe, bestehend aus synthetischem Glimmer, $SiO_2$-Plättchen, $Al_2O_3$-Plättchen und deren Gemische, ausgewählt. Weiter bevorzugt werden die weitgehend transparenten plättchenförmigen synthetischen Substrate aus der Gruppe, bestehend aus $SiO_2$-Plättchen, $Al_2O_3$-Plättchen und deren Gemische, ausgewählt. Weiter bevorzugt werden die weitgehend transparenten plättchenförmigen synthetischen Substrate aus der Gruppe, bestehend aus synthetischem Glimmer, $SiO_2$-Plättchen und deren Gemische, ausgewählt. Weiterhin bevorzugt werden die weitgehend transparenten plättchenförmigen synthetischen Substrate aus der Gruppe, bestehend aus synthetischem Glimmer, $Al_2O_3$-Plättchen und deren Gemische, ausgewählt. Ganz besonders bevorzugt ist synthetischer Glimmer. Weiterhin bevorzugt sind $Al_2O_3$-Plättchen. Ebenfalls bevorzugt sind $SiO_2$-Plättchen.

Synthetische Substrate sind im Unterschied zu natürlichen Substraten wie beispielsweise natürlichem Glimmer im Wesentlichen frei von eingelagerten Fremdionen, die den Farbton verändern können. Weiterhin kann durch die Verunreinigungen die Helligkeit (L*-Wert) deutlich herabgesetzt werden. Dies lässt sich z.B. durch diffuse Farbmessung der jeweiligen Pulverschüttungen mit einem Farbmessgerät CR 310 der Firma Konica Minolta bestimmen.

| Synthetischer Glimmer | L* = 97,6 |
| Natürlicher Glimmer | L* = 83,6 |

In einer weiteren Ausführungsform können die verwendeten weitgehend transparenten plättchenförmigen synthetischen Substrate L*-Werte ≥ 90, bevorzugt ≥ 92 und besonders bevorzugt ≥ 95 aufweisen.

In einer weiteren Ausführungsform können die verwendeten weitgehend transparenten plättchenförmigen synthetischen Substrate einen Eisengehalt von bis zu 0,2 Gew.-% aufweisen. Der Eisengehalt kann in einem Bereich von 0,01 Gew.-% bis 0,2 Gew.-%, bevorzugt in einem Bereich von 0,1 Gew.-% bis 0,19 Gew.-%, und besonders bevorzugt in einem Bereich von 0,12 Gew.-% bis 0,18 Gew.-% liegen. In einer weiteren Ausführungsform können die verwendeten weitgehend transparenten plättchenförmigen synthetischen Substrate einen Eisengehalt von 0,15 Gew.-% bis 0,2 Gew.-% aufweisen.

In einer weiteren Ausführungsform können die verwendeten weitgehend transparenten plättchenförmigen synthetischen Substrate einen Brechungsindex aus einem Bereich von 1,55 bis 1,70, bevorzugt aus einem Bereich von 1,58 bis 1,68 und besonders bevorzugt aus einem Bereich von 1,59 bis 1,65 aufweisen.

Bei einer bevorzugten Ausführungsform weisen die erfindungsgemäßen Perlglanzpigmente mindestens eine hochbrechende Beschichtung auf. Die hochbrechende Beschichtung hat bevorzugt einen Brechungsindex $n_M > 2,0$ und besonders bevorzugt einen Brechungsindex $n_M > 2,2$.

Besonders bevorzugt ist, dass die hochbrechende Beschichtung eine Metalloxidschicht und/oder eine Metallhydroxidschicht und/oder eine Metalloxidhydratschicht aufweist oder ist.

Als hochbrechende Schichten werden bevorzugt hochbrechende Metalloxide, Metallhydroxide und/oder Metalloxidhydrate verwendet. Als Metalloxide werden bevorzugt Metalloxide der Gruppe bestehend aus Titanoxid, Eisenoxid, Ceroxid, Chromoxid, Zinnoxid, Zirkoniumoxid, Kobaltoxid und Mischungen davon verwendet. Anstelle der oder zusätzlich zu den vorstehend angegebenen Oxiden können selbstverständlich auch die entsprechenden Metallhydroxide und/oder Metalloxidhydrate verwendet werden.

Das Titanoxid kann dabei aus der Gruppe, die aus Rutil, Anatas und Brookit, besteht, ausgewählt werden. Vorzugsweise liegt das Titanoxid als $TiO_2$ in der Anatasmodifikation vor.

Das Eisenoxid wird vorzugsweise aus der Gruppe, die aus Hämatit, Goethit und/oder Magnetit besteht ausgewählt. Vorzugsweise liegt das Eisenoxid als $Fe_2O_3$ (Hämatit) und/oder $Fe_3O_4$ (Magnetit) vor.

Besonders bevorzugt sind $TiO_2$ und $Fe_2O_3$ sowie Mischungen und Kombinationen hiervon. In Mischungen dieser Oxide liegt das $TiO_2$ in einer Pseudobrookit- oder Pseudorutilmodifikation oder aber auch als Ilmenit vor.

$TiO_2$ beschichtete Pigmente ermöglichen die Bereitstellung von silbernen Farbtönen. Diese Pigmente sind für den sogenannten "immediate whitening-Effekt" äußerst interessant. Hierunter versteht man kosmetische Hautformulierungen, die der Haut ein weißeres Aussehen verleihen. Bislang werden meist $TiO_2$-Pigmente verwendet, um diesen "immediate whitening-Effekt" zu erzielen. Nachteiligerweise besitzen die herkömmlichen $TiO_2$-Pigmente oftmals einen sogenannten Masking-Effekt. Die erfindungsgemäßen Pigmente ermöglichen aufgrund der Kombination von vorhandenem Glanz und der besonderen Feinteiligkeit einen natürlicheren Effekt.

Im Fall von Eisenoxiden als hochbrechende Beschichtung sind die erfindungsgemäßen Perlglanzpigmente insbesondere in Haarformulierungen vorteilhaft einsetzbar. Derartige Pigmente unterstützen die natürliche Haarfarbe und wirken aufgrund ihrer Feinheit dennoch nicht als "Schuppen". Dies gilt in diesem Fall insbesondere für dunkle Haare, vorzugsweise brünette Haare. Auch blonde Haare können durch kosmetische Haarmittel, die "goldene" oder "beige" erfindungsgemäße Perlglanzpigmente enthalten, in ihrer Farbgebung unterstützt oder verstärkt werden. Weiterhin können rot, blau oder grün gefärbte Haare durch entsprechend gefärbte Perlglanzpigmente in ihrer Farbgebung unterstützt werden.

Als optisch wirksame Beschichtung oder Schicht, können anstelle oder zusätzlich zu der einen oder mehreren hochbrechenden Metalloxidschicht(en) auch eine oder mehrere semitransparente Metallschichten aufgebracht werden. Zur Erzeugung der semitransparenten Metallschichten werden vorzugsweise ein oder mehrere Metalle aufgebracht, die aus der Gruppe, die aus Silber, Gold, Aluminium, Eisen, Magnesium, Chrom, Kupfer, Zink, Zinn, Mangan, Kobalt, Titan, Tantal, Molybdän und Mischungen sowie Legierungen davon besteht, ausgewählt werden.

Gemäß der Erfindung weisen die Perlglanzpigmente eine Metalloxidschicht aus $TiO_2$ und ein Substrat aus synthetischem Glimmer auf. Vorzugsweise besteht folgender Zusammenhang zwischen dem Metalloxid in Gew.-%, bezogen auf das Gesamtgewicht von $TiO_2$ und synthetischem Glimmer, und der mittleren Schichtdicke der $TiO_2$-Beschichtung:

ein Metalloxidgehalt von 30 - 80 Gew.-% bei einer mittleren Metalloxidschichtdicke von über 20 bis 50 nm;
ein Metalloxidgehalt von 50 - 85 Gew.-% bei einer mittleren Metalloxidschichtdicke von über 50 bis 75 nm;
ein Metalloxidgehalt von 59 - 89 Gew.-% bei einer mittleren Metalloxidschichtdicke von über 75 bis 95 nm;
ein Metalloxidgehalt von 66 - 92 Gew.-% bei einer mittleren Metalloxidschichtdicke von über 95 bis 125 nm;
ein Metalloxidgehalt von 69 - 96 Gew.-% bei einer mittleren Metalloxidschichtdicke von über 125 bis 215 nm.

Besonders bevorzugt besteht folgender Zusammenhang zwischen dem $TiO_2$-Gehalt in Gew.-%, bezogen auf das Gesamtgewicht von $TiO_2$ und synthetischen Glimmer, und der mittleren Schichtdicke der $TiO_2$-Beschichtung:

ein $TiO_2$-Gehalt von 35 - 62 Gew.-% bei einer mittleren $TiO_2$-Schichtdicke von über 20 bis 35 nm;
ein $TiO_2$-Gehalt von 40 - 74 Gew.-% bei einer mittleren $TiO_2$-Schichtdicke von über 35 bis 45 nm;
ein $TiO_2$-Gehalt von 45 - 78 Gew.-% bei einer mittleren $TiO_2$-Schichtdicke von über 45 bis 55 nm;
ein $TiO_2$-Gehalt von 50 - 82 Gew.-% bei einer mittleren $TiO_2$-Schichtdicke von über 55 bis 65 nm;

ein $TiO_2$-Gehalt von 55 - 85 Gew.-% bei einer mittleren $TiO_2$-Schichtdicke von über 65 bis 75 nm;

ein $TiO_2$-Gehalt von 60 - 86,5 Gew.-% bei einer mittleren $TiO_2$-Schichtdicke von über 75 bis 85 nm;

ein $TiO_2$-Gehalt von 65 - 88 Gew.-% bei einer mittleren $TiO_2$-Schichtdicke von über 85 bis 95 nm;

ein $TiO_2$-Gehalt von 67 - 89 Gew.-% bei einer mittleren $TiO_2$-Schichtdicke von über 95 bis 105 nm;

ein $TiO_2$-Gehalt von 68 - 90 Gew.-% bei einer mittleren $TiO_2$-Schichtdicke von über 105 bis 115 nm;

ein $TiO_2$-Gehalt von 69 - 91 Gew.-% bei einer mittleren $TiO_2$-Schichtdicke von über 115 bis 125 nm;

ein $TiO_2$-Gehalt von 70 - 92 Gew.-% bei einer mittleren $TiO_2$-Schichtdicke von über 125 bis 135 nm;

ein $TiO_2$-Gehalt von 71 - 92,5 Gew.-% bei einer mittleren $TiO_2$-Schichtdicke von über 135 bis 145 nm;

ein $TiO_2$-Gehalt von 72 - 93 Gew.-% bei einer mittleren $TiO_2$-Schichtdicke von über 145 bis 155 nm;

ein $TiO_2$-Gehalt von 73 - 93 Gew.-% bei einer mittleren $TiO_2$-Schichtdicke von über 155 bis 165 nm;

ein $TiO_2$-Gehalt von 73,5 - 93,5 Gew.-% bei einer mittleren $TiO_2$-Schichtdicke von über 165 bis 175 nm;

ein $TiO_2$-Gehalt von 74 - 94 Gew.-% bei einer mittleren $TiO_2$-Schichtdicke von über 175 bis 185 nm;

ein $TiO_2$-Gehalt von 74,5 - 94 Gew.-% bei einer mittleren $TiO_2$-Schichtdicke von über 185 bis 195 nm;

ein $TiO_2$-Gehalt von 75 - 94,5 Gew.-% bei einer mittleren $TiO_2$-Schichtdicke von über 195 bis 205 nm;

ein $TiO_2$-Gehalt von 75,5 - 95 Gew.-% bei einer mittleren $TiO_2$-Schichtdicke von über 205 bis 215 nm.

Des weiteren besteht vorzugsweise folgender Zusammenhang zwischen dem $TiO_2$-Gehalt in Gew.-%, bezogen auf das Gesamtgewicht von $TiO_2$ und synthetischem Glimmer, und der mittleren Schichtdicke der $TiO_2$-Beschichtung:

ein $TiO_2$-Gehalt aus einem Bereich von 47,5 - 62 Gew.-% bei einer mittleren $TiO_2$-Schichtdicke aus einem Bereich von über 20 bis 35 nm;

ein $TiO_2$-Gehalt aus einem Bereich von 58 - 74 Gew.-% bei einer mittleren $TiO_2$-Schichtdicke aus einem Bereich von über 35 bis 45 nm;

ein $TiO_2$-Gehalt aus einem Bereich von 63 - 78 Gew.-% bei einer mittleren $TiO_2$-Schichtdicke aus einem Bereich von über 45 bis 55 nm;

ein $TiO_2$-Gehalt aus einem Bereich von 67 - 82 Gew.-% bei einer mittleren $TiO_2$-Schichtdicke aus einem Bereich von über 55 bis 65 nm;

ein $TiO_2$-Gehalt aus einem Bereich von 70 - 85 Gew.-% bei einer mittleren $TiO_2$-Schichtdicke aus einem Bereich von über 65 bis 75 nm;

ein $TiO_2$-Gehalt aus einem Bereich von 73,5 - 86,5 Gew.-% bei einer mittleren $TiO_2$-Schichtdicke aus einem Bereich von über 75 bis 85 nm;

ein $TiO_2$-Gehalt aus einem Bereich von 75 - 88 Gew.-% bei einer mittleren $TiO_2$-Schichtdicke aus einem Bereich von über 85 bis 95 nm;

ein $TiO_2$-Gehalt aus einem Bereich von 76,5 - 89 Gew.-% bei einer mittleren $TiO_2$-Schichtdicke aus einem Bereich von über 95 bis 105 nm;

ein $TiO_2$-Gehalt aus einem Bereich von 78,5 - 90 Gew.-% bei einer mittleren $TiO_2$-Schichtdicke aus einem Bereich von über 105 bis 115 nm;

ein $TiO_2$-Gehalt aus einem Bereich von 80 - 91 Gew.-% bei einer mittleren $TiO_2$-Schichtdicke aus einem Bereich von über 115 bis 125 nm;

ein $TiO_2$-Gehalt aus einem Bereich von 81,5 - 92 Gew.-% bei einer mittleren $TiO_2$-Schichtdicke aus einem Bereich von über 125 bis 135 nm;

ein $TiO_2$-Genalt aus einem Bereich von 83 - 92,5 Gew.-% bei einer mittleren $TiO_2$-Schichtdicke aus einem Bereich von über 135 bis 145 nm;

ein $TiO_2$-Gehalt aus einem Bereich von 84 - 93 Gew.-% bei einer mittleren $TiO_2$-Schichtdicke aus einem Bereich von über 145 bis 155 nm;

ein $TiO_2$-Gehalt aus einem Bereich von 85 - 93 Gew.-% bei einer mittleren $TiO_2$-Schichtdicke aus einem Bereich von über 155 bis 165 nm;

ein $TiO_2$-Gehalt aus einem Bereich von 86 - 93,5 Gew.-% bei einer mittleren $TiO_2$-Schichtdicke aus einem Bereich von über 165 bis 175 nm;

ein $TiO_2$-Gehalt aus einem Bereich von 87 - 94 Gew.-% bei einer mittleren $TiO_2$-Schichtdicke aus einem Bereich von über 175 bis 185 nm;

ein $TiO_2$-Gehalt aus einem Bereich von 87,5 - 94 Gew.-% bei einer mittleren $TiO_2$-Schichtdicke aus einem Bereich von über 185 bis 195 nm;

ein $TiO_2$-Gehalt aus einem Bereich von 88 - 94,5 Gew.-% bei einer mittleren $TiO_2$-Schichtdicke aus einem Bereich von über 195 bis 205 nm;

ein $TiO_2$-Geha aus einem Bereich von 89 - 95 Gew.-% bei einer mittleren $TiO_2$-Schichtdicke aus einem Bereich von über 205 bis 215 nm.

Bei einer weiter bevorzugten Ausführungsform besteht vorzugsweise folgender Zusammenhang zwischen dem $TiO_2$-Gehalt in Gew.-%, bezogen auf das Gesamtgewicht von $TiO_2$ und synthetischem Glimmer, und der mittleren Schichtdicke der $TiO_2$-Beschichtung:

ein $TiO_2$-Gehalt aus einem Bereich von 50,5 - 62 Gew.-% bei einer mittleren $TiO_2$-Schichtdicke aus einem Bereich von über 20 bis 35 nm;
ein $TiO_2$-Geha aus einem Bereich von 61 - 74 Gew.-% bei einer mittleren $TiO_2$-Schichtdicke aus einem Bereich von über 35 bis 45 nm;
ein $TiO_2$-Gehalt aus einem Bereich von 65,5 - 78 Gew.-% bei einer mittleren $TiO_2$-Schichtdicke aus einem Bereich von über 45 bis 55 nm;
ein $TiO_2$-Gehalt aus einem Bereich von 69,5 - 82 Gew.-% bei einer mittleren $TiO_2$-Schichtdicke aus einem Bereich von über 55 bis 65 nm;
ein $TiO_2$-Gehalt aus einem Bereich von 72,5 - 85 Gew.-% bei einer mittleren $TiO_2$-Schichtdicke aus einem Bereich von über 65 bis 75 nm;
ein $TiO_2$-Gehalt aus einem Bereich von 75 - 86,5 Gew.-% bei einer mittleren $TiO_2$-Schichtdicke aus einem Bereich von über 75 bis 85 nm;
ein $TiO_2$-Gehalt aus einem Bereich von 77,5 - 88 Gew.-% bei einer mittleren $TiO_2$-Schichtdicke aus einem Bereich von über 85 bis 95 nm;
ein $TiO_2$-Gehalt aus einem Bereich von 79 - 89 Gew.-% bei einer mittleren $TiO_2$-Schichtdicke aus einem Bereich von über 95 bis 105 nm;
ein $TiO_2$-Gehalt aus einem Bereich von 80,5 - 90 Gew.-% bei einer mittleren $TiO_2$-Schichtdicke aus einem Bereich von über 105 bis 115 nm;
ein $TiO_2$-Gehalt aus einem Bereich von 82 - 91 Gew.-% bei einer mittleren $TiO_2$-Schichtdicke aus einem Bereich von über 115 bis 125 nm;
ein $TiO_2$-Gehalt aus einem Bereich von 83 - 92 Gew.-% bei einer mittleren $TiO_2$-Schichtdicke aus einem Bereich von über 125 bis 135 nm;
ein $TiO_2$-Gehalt aus einem Bereich von 84,5 - 92,5 Gew.-% bei einer mittleren $TiO_2$-Schichtdicke aus einem Bereich von über 135 bis 145 nm;
ein $TiO_2$-Gehalt aus einem Bereich von 85,5 - 93 Gew.-% bei einer mittleren $TiO_2$-Schichtdicke aus einem Bereich von über 145 bis 155 nm;
ein $TiO_2$-Gehalt aus einem Bereich von 86,5 - 93 Gew.-% bei einer mittleren $TiO_2$-Schichtdicke aus einem Bereich von über 155 bis 165 nm;
ein $TiO_2$-Gehalt aus einem Bereich von 87 - 93,5 Gew.-% bei einer mittleren $TiO_2$-Schichtdicke aus einem Bereich von über 165 bis 175 nm;
ein $TiO_2$-Gehalt aus einem Bereich von 88 - 94 Gew.-% bei einer mittleren $TiO_2$-Schichtdicke aus einem Bereich von über 175 bis 185 nm;
ein $TiO_2$-Gehalt aus einem Bereich von 88,5 - 94 Gew.-% bei einer mittleren $TiO_2$-Schichtdicke aus einem Bereich von über 185 bis 195 nm;
ein $TiO_2$-Gehalt aus einem Bereich von 89 - 94,5 Gew.-% bei einer mittleren $TiO_2$-Schichtdicke aus einem Bereich von über 195 bis 205 nm;
ein $TiO_2$-Gehalt aus einem Bereich von 89,5 - 95 Gew.-% bei einer mittleren $TiO_2$-Schichtdicke aus einem Bereich von über 205 bis 215 nm.

Ganz besonders bevorzugt besteht folgender Zusammenhang zwischen $TiO_2$-Gehalt in Gew.-%, bezogen auf das Gesamtgewicht von $TiO_2$ und synthetischem Glimmer, und der mittleren Schichtdicke der $TiO_2$-Beschichtung:

ein $TiO_2$-Gehalt von 35 - 62 Gew.-% bei einer mittleren $TiO_2$-Schichtdicke von über 20 bis 35 nm;
ein $TiO_2$-Gehalt von 40 - 74 Gew.-% bei einer mittleren $TiO_2$-Schichtdicke von über 35 bis 45 nm;
ein $TiO_2$-Gehalt von 45 - 78 Gew.-% bei einer mittleren $TiO_2$-Schichtdicke von über 45 bis 55 nm;
ein $TiO_2$-Gehalt von 50 - 82 Gew.-% bei einer mittleren $TiO_2$-Schichtdicke von über 55 bis 65 nm;
ein $TiO_2$-Gehalt von 55 - 85 Gew.-% bei einer mittleren $TiO_2$-Schichtdicke von über 65 bis 75 nm;
ein $TiO_2$-Gehalt von 60 - 86,5 Gew.-% bei einer mittleren $TiO_2$-Schichtdicke von über 75 bis 85 nm;
ein $TiO_2$-Gehalt von 65 - 88 Gew.-% bei einer mittleren $TiO_2$-Schichtdicke von über 85 bis 95 nm;
ein $TiO_2$-Gehalt von 67 - 89 Gew.-% bei einer mittleren $TiO_2$-Schichtdicke von über 95 bis 105 nm;
ein $TiO_2$-Gehalt von 68 - 90 Gew.-% bei einer mittleren $TiO_2$-Schichtdicke von über 105 bis 115 nm;
ein $TiO_2$-Gehalt von 69 - 91 Gew.-% bei einer mittleren $TiO_2$-Schichtdicke von über 115 bis 125 nm;
ein $TiO_2$-Gehalt von 70 - 92 Gew.-% bei einer mittleren $TiO_2$-Schichtdicke von über 125 bis 135 nm;
ein $TiO_2$-Gehalt von 71 - 92,5 Gew.-% bei einer mittleren $TiO_2$-Schichtdicke von über 135 bis 145 nm.

Insbesondere bevorzugt besteht folgender Zusammenhang zwischen dem $TiO_2$-Gehalt in Gew.-%, bezogen auf das Gesamtgewicht von $TiO_2$ und synthetischem Glimmer, und der mittleren Schichtdicke der $TiO_2$-Beschichtung:

ein $TiO_2$-Gehalt von 47,5 - 62 Gew.-% bei einer mittleren $TiO_2$-Schichtdicke von über 20 bis 35 nm;
ein $TiO_2$-Gehalt von 58 - 74 Gew.-% bei einer mittleren $TiO_2$-Schichtdicke von über 35 bis 45 nm;
ein $TiO_2$-Gehalt von 63 - 78 Gew.-% bei einer mittleren $TiO_2$-Schichtdicke von über 45 bis 55 nm;
ein $TiO_2$-Gehalt von 67 - 82 Gew.-% bei einer mittleren $TiO_2$-Schichtdicke von über 55 bis 65 nm;
ein $TiO_2$-Gehalt von 70 - 85 Gew.-% bei einer mittleren $TiO_2$-Schichtdicke von über 65 bis 75 nm;
ein $TiO_2$-Gehalt von 73,5 - 86,5 Gew.-% bei einer mittleren $TiO_2$-Schichtdicke von über 75 bis 85 nm;
ein $TiO_2$-Gehalt von 75 - 88 Gew.-% bei einer mittleren $TiO_2$-Schichtdicke von über 85 bis 95 nm;
ein $TiO_2$-Gehalt von 76,5 - 89 Gew.-% bei einer mittleren $TiO_2$-Schichtdicke von über 95 bis 105 nm;
ein $TiO_2$-Gehalt von 78,5 - 90 Gew.-% bei einer mittleren $TiO_2$-Schichtdicke von über 105 bis 115 nm;
ein $TiO_2$-Gehalt von 80 - 91 Gew.-% bei einer mittleren $TiO_2$-Schichtdicke von über 115 bis 125 nm;
ein $TiO_2$-Gehalt von 81,5 - 92 Gew.-% bei einer mittleren $TiO_2$-Schichtdicke von über 125 bis 135 nm;
ein $TiO_2$-Gehalt von 83 - 92,5 Gew.-% bei einer mittleren $TiO_2$-Schichtdicke von über 135 bis 145 nm.

Die Erfinder haben überraschend festgestellt, dass Perlglanzpigmente, bei denen der Anteil an $TiO_2$ und die Schichtdicke pro synthetischem Glimmersubstrat die vorstehenden Beziehungen erfüllen, einen hervorragenden Softfokuseffekt zeigen und sich außerordentlich gut zur Verwendung in Kosmetika eignen. Diese Perlglanzpigmente zeichnen sich strukturell durch einen sehr hohen $TiO_2$-Gehalt pro Perlglanzpigmentpartikel aus. Im Vergleich zu herkömmlichen Perlglanzpigmentpartikeln ist mithin der Anteil an $TiO_2$, bezogen auf das synthetische Glimmersubstrat, signifikant erhöht.

Efindungsgemäß besteht folgender Zusammenhang zwischen dem $Fe_2O_3$-Gehalt in Gew.-%, bezogen auf das Gesamtgewicht von $Fe_2O_3$ und synthetischem Glimmer, und der mittleren Schichtdicke der $Fe_2O_3$-Beschichtung:

ein $Fe_2O_3$-Gehalt von 47,5 - 72,4 Gew.-% bei einer mittleren $Fe_2O_3$-Schichtdicke von über 35 bis 45 nm;
ein $Fe_2O_3$-Gehalt von 57,5 - 82,4 Gew.-% bei einer mittleren $Fe_2O_3$-Schichtdicke von über 45 bis 55 nm;
ein $Fe_2O_3$-Gehalt von 62,5 - 87,4 Gew.-% bei einer mittleren $Fe_2O_3$-Schichtdicke von über 55 bis 65 nm.

Die erfindungsgemäßen Perlglanzpigmente zeichnen sich strukturell auch durch einen sehr hohen $Fe_2O_3$-Gehalt pro Perlglanzpigment aus.

In einer weiteren Ausführungsform besitzen die Perlglanzpigmente einen Gesamtbleigehalt $\leq$ 10 ppm, bevorzugt aus einem Bereich von 0,0 ppm bis $\leq$ 9 ppm, weiter bevorzugt aus einem Bereich von 0,0 ppm bis $\leq$ 8 ppm, noch weiter bevorzugt aus einem Bereich von 0,1 ppm bis $\leq$ 7 ppm und besonders bevorzugt aus einem Bereich von 0,1 ppm bis $\leq$ 6,5 ppm.

Die Bestimmung des Bleigehalts erfolgt hierbei über Feststoff-Graphitrohr-Atomabsorptionsspektrometrie. Als Gerät wird vorzugsweise ein ZEENIT 650 mit Feststoffprobengeber SSA 600 (Hersteller: Analytik Jena) eingesetzt.

Verunreinigungen durch Schwermetalle in kosmetischen Formulierungen sind im Interesse des Verbrauchers unerwünscht. Insbesondere erhöhte Bleigehalte in kosmetischen Formulierungen sind in jüngster Zeit in Kritik geraten. Farbadditive werden bzgl. ihres Bleigehalts von der FDA überwacht und dürfen einen Grenzwert von 20 $\mu$g/g nicht überschreiten. Weitere kosmetische Inhaltsstoffe unterliegen im Hinblick auf den Bleigehalt der Verantwortung der Hersteller (Nancy M. Hepp, William R. Mindak, John Cheng, J. Cosmet. Sci., 60, 405 - 414 (Juli / August 2009)).

Als Softfokuseffekt bezeichnet man die Eigenschaft von geeigneten Partikeln, Unebenheiten der menschlichen Haut sowie den Kontrast optisch zu reduzieren und Falten optisch zu glätten. Geeignete Partikel werden in kosmetische Präparate eingearbeitet und als kosmetische Schicht, beispielsweise auf die Haut, aufgetragen. Der Softfokuseffekt tritt auf, wenn das einfallende Licht nach dem Durchgang der kosmetischen Schicht, durch Wechselwirkung mit den auf der Hautoberfläche angeordneten Partikeln, diffus gestreut wird.

Hautfehlstellen wie "Krähenfüße" oder Falten treten nur in Erscheinung, wenn diese einen Kontrast mit dem Hintergrund aufzeigen. Hautfalten wirken wie Lichtfallen, in welchen das auftreffende Licht innerhalb der Falte so lange reflektiert wird, bis durch diese mehrfache Reflexion das Licht nahezu vollständig absorbiert wird.

Im Gegensatz zur hellen umgebenden Haut werden somit die Falten vom Beobachter als nicht reflektierende, dunkle Stellen wahrgenommen.

Durch die Verwendung von stark streuenden Partikeln, wie z.B. feinen Kugeln, wird das Licht vor dem Auftreffen auf die Haut diffus gestreut, so dass die darunter liegenden Hautfehlstellen nahezu unsichtbar werden.

Um jedoch ein natürliches Erscheinungsbild zu erhalten, muss neben einer maximalen Streuintensität auch eine hohe Lichttransmission durch die Partikel gewährleistet sein. Diese bedingt, dass der natürliche Hautfarbton nicht verändert bzw. überdeckt wird, d.h. der natürliche Teint bleibt für den Betrachter weiterhin sichtbar.

Um einen Softfokuseffekt zu erzielen, müssen die eingesetzten Partikel folgende Rahmenbedingungen erfüllen:

a) eine maximale diffuse Reflexion
b) eine minimale gerichtete Reflexion
c) eine möglichst hohe Transmission.

Die erfindungsgemäßen, mit $TiO_2$ beschichteten Perlglanzpigmente eignen sich auch hervorragend als UV-Absorber. Unter UV-Absorption ist der gesamte Lichtverlust, welcher beim Durchgang durch eine UV-Absorber enthaltende Schicht resultiert, zu verstehen. Dieser Lichtverlust setzt sich aus der Gesamtreflexion sowie der Gesamtabsorption zusammen.
$TiO_2$-Schichten sind bekanntlich stark UV-reflektierend und daher werden Perlglanzpigmente u.a. auch als UV-Absorber eingesetzt. Die erfindungsgemäßen Perlglanzpigmente sind aufgrund des hohen $TiO_2$-Gehaltes besonders gut als UV-Absorber geeignet. Weiterhin bewirkt möglicherweise der hohe Kantenanteil der feinen Pigmente eine hohe UV-Absorption.

Gemäß einer bevorzugten Weiterbildung der Erfindung weisen die Perlglanzpigmente auf der optisch wirksamen Schicht, vorzugsweise hochbrechenden Schicht, mindestens eine weitere Schutzschicht auf.

Die mindestens eine weitere Schutzschicht kann dabei mindestens eine Metalloxidschicht, deren Metalloxide aus der Gruppe, die aus $SiO_2$, $Al_2O_3$, Cer-oxid, Mischungen und / oder Kombinationen davon, besteht, ausgewählt werden. Als Schutzschicht kann auch eine Kunststoffbeschichtung, beispielsweise Polyacrylatschicht, aufgebracht werden.

Besonders bevorzugt sind hierbei Schutzschichten aus $SiO_2$ oder aus Ceroxid in Kombination mit $SiO_2$, wie sie in den EP 1 727 864 A1 und EP 1 682 622 A1 beschrieben sind.

Bei einer weiteren Ausführungsform umfasst die vorliegende Erfindung ein Perlglanzpigment basierend auf synthetischem Glimmer mit einer Dichte $\rho_s$ und mit mindestens einer optisch wirksamen Beschichtung mit einer Dichte $\rho_M$, wobei der synthetische Glimmer eine mittlere Größe $d_{50}$ aus einem Bereich von 2,1 $\mu$m bis 7,0 $\mu$m, eine mittlere Höhe $h_s$ aus einem Bereich von 40 nm bis 100 nm aufweist und der Gesamtbleigehalt der Perlglanzpigmente bei 0,0 ppm bis $\leq$ 9 ppm liegt.

Bei einer weiteren Ausführungsform umfasst die vorliegende Erfindung ein Perlglanzpigment basierend auf synthetischem Glimmer mit einer Dichte $\rho_s$ und mit mindestens einer optisch wirksamen Beschichtung mit einer Dichte $\rho_M$, wobei der synthetische Glimmer eine mittlere Größe $d_{50}$ aus einem Bereich von 2,0 $\mu$m bis 8,0 $\mu$m, eine mittlere Höhe $h_s$ aus einem Bereich von 40 nm bis 110 nm aufweist und die Perlglanzpigmente eine Größenverteilung mit einem $d_{90}$-Wert aus einem Bereich von 5,0 $\mu$m bis 11,0 $\mu$m besitzen, wobei der Gesamtbleigehalt der Perlglanzpigmente bei 0.0 ppm bis $\leq$ 9 ppm liegt.

Bei einer weiteren Ausführungsform umfasst die vorliegende Erfindung ein Perlglanzpigment basierend auf synthetischem Glimmer mit einer Dichte $\rho_s$ und mit mindestens einer optisch wirksamen Beschichtung mit einer Dichte $\rho_M$, wobei der synthetische Glimmer eine mittlere Größe $d_{50}$ aus einem Bereich von 2,0 $\mu$m bis 8,0 $\mu$m, eine mittlere Höhe $h_s$ aus einem Bereich von 40 nm bis 110 nm, einen L*-Wert $\geq$ 90 aufweist und die Perlglanzpigmente eine Größenverteilung mit einem $d_{90}$-Wert aus einem Bereich von 5,0 $\mu$m bis 11,0 $\mu$m besitzen, wobei der Gesamtbleigehalt der Perlglanzpigmente bei 0,0 ppm bis $\leq$ 9 ppm liegt.

Bei einer weiteren Ausführungsform umfasst die vorliegende Erfindung eine kosmetische Formulierung enthaltend Perlglanzpigmente basierend auf synthetischem Glimmer mit einer Dichte $\rho_s$ und mit mindestens einer optisch wirksamen Beschichtung mit einer Dichte $\rho_M$, wobei der synthetische Glimmer eine mittlere Größe $d_{50}$ aus einem Bereich von 2,0 $\mu$m bis 8,0 $\mu$m und eine mittlere Höhe $h_s$ aus einem Bereich von 40 nm bis 110 nm aufweist und der Gesamtbleigehalt der Perlglanzpigmente bei 0,0 ppm bis $\leq$ 9 ppm liegt.

Bei einer weiteren Ausführungsform umfasst die vorliegende Erfindung eine kosmetische Formulierung enthaltend Perlglanzpigmente basierend auf synthetischem Glimmer mit einer Dichte $\rho_s$ und mit mindestens einer optisch wirksamen Beschichtung mit einer Dichte $\rho_M$, wobei der synthetische Glimmer eine mittlere Größe $d_{50}$ aus einem Bereich von 2,5 $\mu$m bis 6,0 $\mu$m und eine mittlere Höhe $h_s$ aus einem Bereich von 40 nm bis 95 nm aufweist und der Gesamtbleigehalt der Perlglanzpigmente bei 0,0 ppm bis $\leq$ 8 ppm liegt.

Bei einer weiteren Ausführungsform umfasst die vorliegende Erfindung eine kosmetische Formulierung enthaltend Perlglanzpigmente basierend auf synthetischem Glimmer mit einer Dichte $p_s$ und mit mindestens einer optisch wirksamen Beschichtung mit einer Dichte $\rho_M$, wobei der synthetische Glimmer eine mittlere Größe $d_{50}$ von 2,0 $\mu$m bis 8,0 $\mu$m und eine mittlere Höhe $h_s$ aus einem Bereich von 40 nm bis 110 nm aufweist und die gesamte kosmetische Formulierung einen Bleigehalt von $\leq$ 15 ppm besitzt.

Bei einer weiteren Ausführungsform umfasst die vorliegende Erfindung eine Tintenstrahltinte enthaltend Perlglanzpigmente basierend auf synthetischem Glimmer mit einer Dichte $p_s$ und mit mindestens einer optisch wirksamen Beschichtung mit einer Dichte $\rho_M$, wobei der synthetische Glimmer eine mittlere Höhe $h_s$ aus einem Bereich von 40 nm bis 110 nm besitzt und die Perlglanzpigmente eine Größenverteilung mit einem $d_{90}$-Wert aus einem Bereich von 5,0 $\mu$m bis 11,0 $\mu$m aufweisen.

Das erfindungsgemäße Verfahren zur Herstellung der erfindungsgemäßen Perlglanzpigmente umfasst folgende Schritte:

a) Klassieren des weitgehend transparenten plättchenförmigen synthetischen Substrates unter Erhalt eines Substrates mit einer mittleren Höhe $h_s$ aus einem Bereich von 40 nm bis 110 nm,

b) Beschichten des klassierten weitgehend transparenten plättchenförmigen synthetischen Substrates mit mindestens einer optisch wirksamen, vorzugsweise hochbrechenden Schicht unter Erhalt eines Perlglanzpigmentes mit einer mittleren Größe $d_{50}$ aus einem Bereich von 2,1 μm bis 8,6 μm.

Alternativ können die weitgehend transparenten plättchenförmigen synthetischen Substrate zuerst mit mindestens einer optisch wirksamen, vorzugsweise hochbrechenden Schicht beschichtet werden und die so erhaltenen Perlglanzpigmente anschließend nach Größe klassiert werden.

Vorzugsweise erfolgt das Beschichten der weitgehend transparenten plättchenförmigen synthetischen Substrate in Schritt b) nach dem Größenklassieren in Schritt a).

Das Klassieren des weitgehend transparenten, vorzugsweise transparenten, plättchenförmigen synthetischen Substrates kann dabei mittels verschiedener Methoden wie Sedimentation im Schwerefeld, Sedimentation im Dekanter, Sieben, Verwendung eines Zyklons oder Hydrozyklons, Spiralklassifikation oder einer Kombination von zwei oder mehreren dieser Methoden erfolgen. Dabei kann eine Methode wie z.B. Sieben auch in mehreren aufeinanderfolgenden Schritten verwendet werden.

Vorzugsweise werden die erfindungsgemäßen Perlglanzpigmente in Beschichtungsmitteln, die vorzugsweise aus der Gruppe, die aus Lacken, Druckfarben, Tintenstrahltinten, Tonern, Kosmetika, Kunststoffen, Textilien, Glas, Email und Keramik besteht, ausgewählt werden, verwendet.

Vorzugsweise ist das erfindungsgemäße Beschichtungsmittel ein Kosmetikum, das aus der Gruppe, die aus Abdeckstiften, Körperpuder, Gesichtspuder, gepresstem und losem Puder, Gesichtsmakeup, Pudercreme, Crememakeup, Emulsionsmakeup, Wachsmakeup, Foundation, Moussemakeup, Wangenrouge, Augenmakeup wie Lidschatten, Mascara, Eyelinern, flüssigen Eyelinern, Augenbrauenstiften, Lippenpflegestiften, Lippenstiften, Lip Gloss, Lip Linern, Haarstylingkompositionen wie Haarspray, Haarfestiger, Haarmousse, Haargel, Haarwachs, Haarmascara, permanenten und semi-permanenten Haarfarben, temporären Haarfarben, Hautpflegekompositionen wie Lotions, Gele, Emulsionen sowie Nagellackkompositionen besteht, ausgewählt wird.

Vorzugsweise werden die erfindungsgemäßen Perlglanzpigmente als Softfokus Pigment verwendet, insbesondere in Kosmetika.

Das erfindungsgemäße Beschichtungsmittel ist vorzugsweise ein kosmetisches Präparat, enthaltend eines der erfindungsgemäßen Perlglanzpigmente. Bei dem erfindungsgemäßen kosmetischen Präparat kann es sich dabei um die obenstehend genannten Kosmetika handeln.

In einer weiteren Ausführungsform kann die kosmetische Formulierung einen Bleigehalt $\leq 15$ ppm aufweisen, bevorzugt kann der Bleigehalt in einem Bereich von 0,0 ppm bis 13 ppm, weiter bevorzugt kann der Bleigehalt in einem Bereich von 1,0 ppm bis 11 ppm und besonders bevorzugt kann der Bleigehalt in einem Bereich von 2,0 ppm bis 10 ppm liegen.

Die erfindungsgemäßen Perlglanzpigmente lassen sich auch vorteilhaft in Abmischungen mit transparenten und deckenden Weiß-, Bunt- und Schwarzpigmenten sowie weiteren Effektpigmenten, wie beispielsweise Metalleffektpigmenten verwenden.

Die Bestimmung der mittleren Schichtdicken und deren Verteilung bzw. Standardabweichung eines Substrates kann - wie im Stand der Technik bekannt - mit Hilfe von REM-Messungen erfolgen. Dazu werden die Perlglanzpigmente in einen Lack einbracht, beispielsweise durch Spritzung oder Rakelabzug, auf einen Untergrund, beispielsweise Blech oder Pappe, aufgebracht und ausgehärtet. Anschließend wird ein Querschliff am ausgehärteten Lack durchgeführt und dieser Querschliff wird im REM untersucht und die Pigmentteilchen werden vermessen. Um statistisch gesicherte Werte zu erhalten, sollten mindestens 100 Pigmentteilchen gezählt werden. Im Rahmen dieser Erfindung kann die Bestimmung der Schichtdicke des Substrates und der optisch wirksamen Schicht, beispielsweise einer Metalloxidbeschichtung oder einer semitransparenten Metallschicht, durch diese Methode erfolgen.

Bei dieser Methode ist darauf zu achten, dass die Perlglanzpigmente weitgehend planparallel orientiert sind. Hierunter versteht man, dass etwa 90 % der Perlglanzpigmente nicht mehr als $\pm$ 15° und bevorzugt nicht mehr als $\pm$ 10° von der mittleren Orientierung abweichen.

Bei einer schlechten Orientierung der Perlglanzpigmente im Lackfilm erhält man einen signifikanten Messfehler. Dieser ist einerseits dadurch bedingt, dass die Perlglanzpigmente im Querschliff um einen azimuthalen Winkel $\alpha$ zum Beobachter gekippt sind. Andererseits erhält man aufgrund des umgebenden Bindemittelmediums keine Tiefenschärfe der Abbildung, so dass dieser Winkel nicht abschätzbar ist. Mithin "sieht" man ein Bild der Schichtdicke, das um den Faktor $1/\cos\alpha$ vergrößert ist. Dieser Faktor verursacht bei größeren Winkeln einen signifikanten Fehler. In Abhängigkeit von der Größe des Winkels $\alpha$ können die mit dieser Methode bestimmten Schichtdicken daher zu hoch sein.

Vorzugsweise wird im Rahmen dieser Erfindung die mittlere Substratschichtdicke $h_s$ gemäß dem nachfolgend

beschriebenen Verfahren bestimmt, um zu exakteren Ergebnissen zu kommen. Bei dem Verfahren wird die mittlere Substratdicke aus dem Zusammenhang zwischen Metalloxidgehalt und der Schichtdicke des Metalloxides bestimmt. Feinere und, wie im Folgenden gezeigt wird, vor allem dünnere Substrate besitzen höhere spezifische Oberflächen. Wenn diese dünneren Substrate mit einem Material beschichtet werden, so müssen sie, damit die Beschichtung eine bestimmte Schichtdicke erreicht, mit mehr Material als dickere Substrate (pro Gewichtseinheit) beschichtet werden. Dies äußert sich in einem höheren spezifischen Gehalt des Beschichtungsmaterials am Gesamtperlglanzpigment, d.h. einem höheren Gehalt an Beschichtungsmaterial, bezogen auf das Gewicht an eingesetztem Substrat.

Dem Verfahren liegt folgendes Modell zugrunde:

a) die Pigmente bestehen aus Zylindern (Plättchen) mit einem einheitlichen Radius $r_s$ und einer einheitlichen Höhe $h_s$. Es wird folglich von vornherein mit "Mittelwerten" gerechnet.

b) Die Wahrscheinlichkeit, dass sich die Beschichtungsmoleküle auf dem Substrat abscheiden, ist überall gleich hoch. Mithin besteht kein Unterschied beispielsweise zwischen Rand oder Deckfläche der Plättchenschichtdicke. Als Folge dieser Annahme bildet sich überall eine einheitliche Schichtdicke $d_M$ der Beschichtung aus. Der Index M steht hier für optische wirksame Beschichtung, vorzugsweise Metalloxid und/oder Metall. Die einheitliche Beschichtungsdicke wird bei REM-Untersuchungen an vielen beschichteten plättchenförmigen Effektpigmenten tatsächlich beobachtet.

c) Nebenfällungen von M werden vernachlässigt, d.h. alles Material von M wird als Beschichtung auf dem Substrat aufgebracht.

Der Gehalt an der Beschichtung M wird folgendermaßen definiert:

$$c_M = 100 * \frac{m_M}{m_M + m_S} \qquad \text{(Gl. 1)}$$

Dabei ist $m_M$ die Masse der Beschichtung und $m_S$ die Masse des Substrates. Diese lassen sich auch über die Dichten und Volumina ausdrücken:

$$c_M = 100 * \frac{\rho_M \cdot V_M}{\rho_M \cdot V_M + \rho_S \cdot V_S} \qquad \text{(Gl. 2)}$$

Hierbei sind $\rho_S$ und $\rho_M$ die Dichten des Substrates und der Beschichtung. Für das Volumen des Substrates gilt folgende einfache Beziehung (Zylindervolumen):

$$V_S = \pi\, r_S^2\, h_S \qquad \text{(Gl. 3)}$$

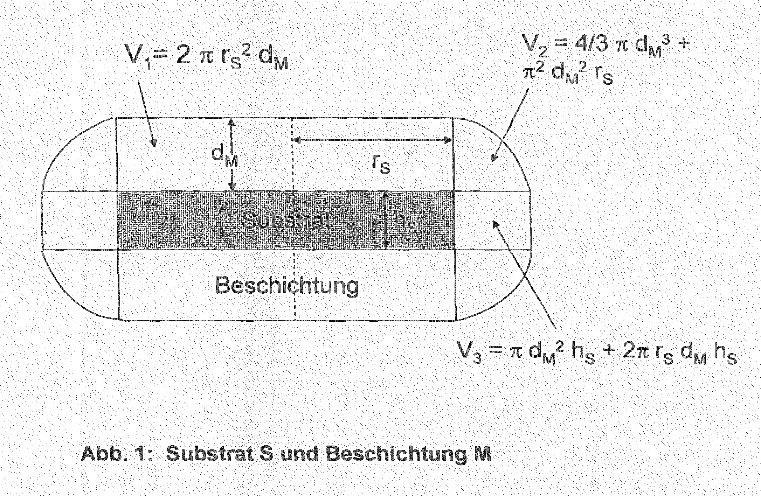

Die Berechnung des Volumens des Beschichtungsmaterials $V_M$ erfolgt nach einem Modell, welches in Abbildung 1 skizziert ist.

Das Volumen des abgeschiedenen Metalloxides teilt sich dabei prinzipiell zwischen den Stirnflächen und dem Rand auf und wird in drei Termen dargestellt (s. Abb. 1):

$$V_M = (V_{M,1} + V_{M,2} + V_{M,3}) \qquad \text{(Gl. 4)}$$

$$V_M = \left[ 2\pi \cdot d_M (r_S)^2 + \left(\frac{4}{3}\pi \cdot d_M^3 + \pi^2 d_M^2 r_S\right) + \left(\pi \cdot d_M^2 h_S + 2\pi \cdot r_S \cdot d_M \cdot h_S\right) \right] \qquad \text{(Gl. 5)}$$

Hierbei ist $h_S$ die mittlere Höhe des Substrates, $r_S$ der mittlere Durchmesser des Substrates und $d_M$ die geometrische Höhe der Schichtdicke des Metalloxides.

Kombiniert man diese Gleichungen, so erhält man schließlich folgenden Ausdruck:

$$c_M = \frac{100}{1 + \frac{\rho_S}{\rho_M} * \frac{h_S \cdot r_S^2}{\left(\frac{4}{3}d_M^3 + (\pi \cdot r_S + h_S) \cdot d_M^2 + (2r_S^2 + 2r_S h_S) \cdot d_M\right)}} \qquad (Gl. 6)$$

Löst man diese Gleichung wiederum nach der mittleren Substratdicke $h_S$ auf, so erhält man den folgenden Ausdruck:

$$h_S = \frac{\frac{4d_M^3}{3r_S^2} + \frac{\pi d_M^2}{r_S} + 2 \cdot d_M}{\frac{\rho_S}{\rho_M \cdot (\frac{100}{c_M} - 1)} - \left(\frac{d_M}{r_S}\right)^2 - 2 \cdot \frac{d_M}{r_S}} \qquad (Gl. 7)$$

Im Rahmen dieser Erfindung wird die mittlere Substratdicke $h_S$ bevorzugt über diese Gleichung definiert, wenn die geometrische Schichtdicke $d_M$ 40 nm bis 180 nm ist. Bei höheren Schichtdicken ist die Formel ungenau, da aufgrund des hohen Gehaltes an dem optisch aktiven Beschichtungsmaterial der Gehalt $c_M$ einem Grenzwert zuläuft. Bei niedrigen Schichtdicken ist ebenfalls keine gute Differenzierung möglich.

In einer weiteren Ausführungsform ist der Schichtaufbau der Perlglanzpigmente auch beispielsweise anhand von Querschliffen und/oder durch ESCA (Electron Spectroscopyfor Chemical Analysis) in Verbindung mit Sputterprofilen analysierbar. Liegt die geometrische Schichtdicke $d_M$ außerhalb des angegebenen Bereichs von 40 nm bis 180 nm, so werden die Schichtdicken bevorzugt anhand oben beschriebener Querschliffe bestimmt.

Der mittlere Radius des Substrates wird dabei bevorzugt über Laserbeugungsmessungen an den Perlglanzpigmenten bestimmt, vorzugsweise durch Lasergranulometrie mittels Cilas 1064 der Fa. Quantachrome. Dabei wird der $d_{50}$-Wert der Größensummenverteilungskurve herangezogen; es gilt dann die Beziehung:

$$d_{50}/2 = r_S \qquad (Gl. 8)$$

Die Größe $c_M$ wird über analytische Messungen bestimmt. Hierbei wird bevorzugt eine RFA-Analyse (Röntgenfluoreszenzanalyse) anhand von fein verteiltem Pigmentmaterial durchgeführt. Das Pigmentpulver wird gegebenenfalls zuvor in einer Mühle oder einem Mörser zerkleinert, um ein einheitliches Probenmaterial bereitzustellen. Alternativ kann das Perlglanzpigment auch beispielsweise durch Flußsäure aufgelöst und die RFA-Analyse anschließend aus der Lösung vorgenommen werden.

Weiterhin können die analytischen Gehalte an Substrat und optisch aktivem Material auch über ICP (Inductively Coupled Plasma) bestimmt werden.

Für die Dichten werden bevorzugt Literaturwerte (Handbook Chemistry and Physics) verwendet. Übliche Werte sind beispielsweise:

Tabelle 1: Dichten üblicher Materialien von Perlglanzpigmenten

| Material | Dichte | Funktion |
|---|---|---|
| Synthetischer Glimmer | 2.8 | Substrat |
| $Al_2O_3$ | 4.0 | Substrat (überwiegend) |
| $SiO_2$ | 2.2 - 2.7 | Substrat (überwiegend) |
| $TiO_2$ (Rutil) | 4.3 | Beschichtung |

(fortgesetzt)

| Material | Dichte | Funktion |
|---|---|---|
| $TiO_2$ (Anatas) | 3.9 | Beschichtung |
| $Fe_2O_3$ (Hämatit) | 5.2 | Beschichtung |
| $Fe_3O_4$ (Magnetit) | 5.2 | Beschichtung |

Werden Mischschichten aus zwei oder mehreren hochbrechenden Schichten verwendet, so kann man die Dichte der Beschichtung aus den Literaturwerten gewichtet mit den analytisch zugänglichen Gewichtsverhältnissen der einzelnen Materialien berechnen.

Die Schichtdicke des Metalloxides schließlich kann beispielsweise und bevorzugt über die Farbe des Perlglanzpigmentes bestimmt werden. Die grundlegenden physikalischen Formeln der Optik von Perlglanzpigmenten sind in C. Schmidt, M. Fritz "Optical Physics of Synthetic Interference Pigments" Kontakte (Darmstadt) 1992 (2) S. 15 - 24 dargelegt worden.

Hierbei kann die Farbe auch durch ein geeignetes Rechenprogramm wie die Software "Filmstar" der Fa. FTG Software Associates, USA bestimmt werden. Dabei müssen die optischen Konstanten (Brechzahl n und gegebenenfalls Absorptionskonstante k) der optisch wirksamen Schicht im Bereich der optischen Wellenlängen (400 bis 800 nm) verwendet werden. Derartige Werte sind für die gängigen Materialien gut bekannt.

Weiterhin kann die Schichtdicke anhand der Farbe aus den öffentlich zugänglichen Informationen bestimmt werden. Beispielsweise gibt es bei mit $TiO_2$ beschichteten Perlglanzpigmenten auf Basis von synthetischem Glimmer folgenden bekannten Zusammenhang:

Tabelle 2: Typische Farben und geometrische Schichtdicken von Perlglanzpigmenten

| | Belegung/ geometrische Schichtdicke | Farbe |
|---|---|---|
| Silberweiße Perlglanzpigmente | $TiO_2$: 40 - 60 nm | silber |
| Interferenzpigmente | $TiO_2$: 20-40 nm | fahlblau |
| | $TiO_2$: 60 - 80 nm | gelb |
| | $TiO_2$: 80 -100 nm | rot |
| | $TiO_2$: 100 - 140 nm | blau |
| | $TiO_2$: 120 - 160 nm | grün |
| | $TiO_2$: 280 - 320 nm | grün (III. Ordnung) |
| Farbglanzpigmente | $Fe_2O_3$: 35-45 nm | bronze |
| | $Fe_2O_3$: 45 - 55 nm | kupfer |
| | $Fe_2O_3$: 55 - 65 nm | rot |
| | $Fe_2O_3$: 65 - 75 nm | rotviolett |
| | $Fe_2O_3$: 75 - 85 nm | rotgrün |

In den meisten Fällen wird die Farbe fast ausschließlich durch die Schichtdicke der hochbrechenden Beschichtung bestimmt (F. Hofmeister, Farbe + Lack 95, 557 (1989)).

Insbesondere bei Perlglanzpigmenten mit einer großen Standardabweichung der Substratdickenverteilung hängt die Farbgebung weitgehend nicht von der mittleren Dicke des Substrates ab, sondern ist weitgehend durch die Schichtdicke der hochbrechenden Schicht bestimmt.

Sollte das Substrat mit seiner Schichtdicke ebenfalls in nicht vernachlässigbarer Weise die Interferenzfarbe bestimmen, so muss eine genauere optische Rechnung herangezogen werden. Dabei lässt sich die Schichtdicke des Substrates wie auch der optisch aktiven Schicht, vorzugsweise der hochbrechenden Metalloxidschicht z.B. anhand der Lagen des Maxima und/oder Minima des Remissionsspektrums der Perlglanzpigmente bestimmen.

Sollte das Perlglanzpigment eine Mischbeschichtung von zwei oder mehreren hochbrechenden Oxiden aufweisen, so sind die optischen Konstanten analog zur Dichteberechnung aus einer Gewichtung aus den analytisch zugänglichen Gewichtsverhältnissen der einzelnen hochbrechenden Oxide zu berechnen.

Weist das Perlglanzpigment dagegen eine Kombination zweier hochbrechender Oxide auf, so lässt sich das Modell dennoch verwenden. Bei der ersten Beschichtung mit hochbrechendem Metalloxid kann Gl. 7 direkt verwendet werden. Bei der Berechnung des zweiten hochbrechenden Oxides ist jedoch die Schichtdicke des ersten Oxides zu berücksichtigen.

Weiterhin kann die Schichtdicke der optischen wirksamen Schicht, bevorzugt der hochbrechenden Metalloxidschicht auch durch die REM-Auszählung an gut orientierten Querschliffen der Perlglanzpigmente bestimmt werden.

Eine weitere Methode zur Bestimmung der mittleren Substratschichtdicke besteht darin, die Dicken der (beschichteten) Perlglanzpigmente gemäß der in der WO 2004/087816 A2 beschrieben Methode zu präparieren und ebenfalls im REM zu vermessen. Dabei sollten mindestens 100 Pigmentteilchen vermessen werden, um eine aussagefähige Statistik zu erhalten. Anschließend ermittelt man den arithmetischen Mittelwert. Dieser stellt die mittlere Dicke des Perlglanzpigmentes $d_{tot}$ dar und es gilt natürlicherweise:

$$d_{tot} = 2\,d_M + h_S \qquad (Gl.\ 9)$$

Man kann ausgehend von Gleichung (7) mittels Gleichung 9 $d_M$ eliminieren und dann nach $h_s$ auflösen. Dabei kann man in guter Näherung die höheren Terme von $h_s$ vernachlässigen und so $h_s$ aus dem Zusammenhang des Gehaltes der optisch aktiven Schicht $c_M$ und der mittleren Pigmentgesamtschichtdicke $d_{tot}$ ermitteln.

Das auf Gl. 7 basierende Verfahren zur Bestimmung der mittleren Substratschichtdicke kann auch allgemein auf plättchenförmige Effektpigmente verwendet werden. Diese besitzen ein plättchenförmiges Substrat sowie einer Beschichtung. Das plättchenförmige Substrat umfasst dabei auch Metallpigmente.

In weiteren erfindungsgemäßen Ausführungsformen weisen die Perlglanzpigmente mindestens eine weitere niedrigbrechende Schicht auf. Diese Schicht kann zwischen Substrat und hochbrechender Schicht oder auf der hochbrechenden Schicht angebracht sein.

Die nachfolgenden Beispiele erläutern die Erfindung ohne sie jedoch zu beschränken.

## Beispiele 1 a-c: (TiO$_2$-beschichteter synthetischer Glimmer)

### Beispiel 1α: Klassierung synthetischer Glimmer

1 kg plättchenförmiger synthetischer Glimmer der Fraktion 10 - 40 $\mu$m (Fa. Shantou F.T.Z. Sanbao Pearl Luster Mica Tech Co., Ltd. China) wurden mit 1000 mL VE-Wasser (VE = vollentsalzt) versetzt und dann in einem Laborkoller der Fa. American Cyanamid Company ca. 30 min delaminiert.

Die erhaltene Paste wurde anschließend mit VE-Wasser auf 10 Gew.-% Feststoffgehalt gebracht und dann in einem Labordissolver der Fa. Pendraulik des Typs TD 200 45 min behandelt. Hierbei wurde darauf geachtet, dass durch Kühlung die Temperatur der Suspension 80°C nicht überstieg.

Die Glimmersuspension wurde dann mit VE-Wasser auf 3 Gew.-% Feststoffgehalt verdünnt und über ein Sedimentationsgefäß 5 h absedimentiert. Der Überstand wurde abgesaugt, und der Bodensatz erneut mit Wasser aufgenommen, kräftig aufgerührt und erneut 5 h absedimentiert. Dieser Vorgang wurde insgesamt viermal wiederholt, bis nahezu kein Überstand mehr zu erkennen war.

Das Sedimentationsgefäß besaß eine zylindrische Form mit den Maßen: d= 50 cm; h= 50 cm.

Der aus den Überständen stammende plättchenförmige synthetische Glimmer wurde in einem großen Behältnis gesammelt und durch Zugabe von NaCl zum Absetzen gebracht. Nach ca. 48 h wurde die überstehende klare Salzlösung abgesaugt und der erhaltene Filterkuchen als Ausgangsmaterial für weitere Beschichtungen verwendet.

Auf diese Weise erhielt man einen äußerst feinen plättchenförmigen synthetischen Glimmer mit einem $d_{50}$ = 3,4 $\mu$m (Cilas 1064) der volumengemittelten Teilchengrößenverteilung sowie einer mittleren Dicke $h_s$ (aus REM) = 79 nm.

### Beispiel 1a: Interferenz silber

100 g plättchenförmiger synthetischer Glimmer aus Beispiel 1α (Feststoffgehalt: 42,3 Gew.-%) wurden in 200 mL VE-Wasser suspendiert. Durch Zudosieren von verdünnter Salzsäure wurde ein pH-Wert von 2,2 eingestellt und die Suspension auf 80°C erhitzt. Dann wurden 50 ml einer Zinnchloridlösung mit c(Sn) = 24 g/L innerhalb 90 min zudosiert. Durch gleichzeitiges Einleiten einer 15 Gew.-%igen Erdalkalilauge wurde der pH-Wert konstant bei 2,2 gehalten. Nach etwa 1/4 h Unterbrechung, während dieser weiter gerührt wurde, stellte man den pH-Wert der Lösung durch Zudosieren von verdünnter Salzsäure auf 1,8 ein. Anschließend begann man mit der Zugabe von 2,4 L einer Lösung von 150 g

$TiCl_4$ und 50 g HCl pro Liter. Durch gleichzeitiges Einleiten einer 15 Gew.-%igen Erdalkalilauge wurde der pH-Wert konstant gehalten.

Am Ende der Zugabe erhielt man ein silberfarbenes Perlglanzpigment. Die Suspension wurde nach 1 h Nachrühren abgekühlt, über einen Büchnertrichter gesaugt und mit VE-Wasser nahezu ionenfrei gewaschen.

Zuletzt wurde das Perlglanzpigment 20 Minuten lang bei 800°C kalziniert.

**Beispiel 1 b: Interferenz rot**

100 g plättchenförmiger synthetischer Glimmer aus Beispiel 1$\alpha$ (Feststoffgehalt: 42,3 Gew.-%) wurden in 200 mL VE-Wasser suspendiert. Durch Zudosieren von verdünnter Salzsäure wurde ein pH-Wert von 2,2 eingestellt und die Suspension auf 80°C erhitzt. Dann wurden 50 mL einer Zinnchloridlösung mit c(Sn) = 24 g/L innerhalb 90 min zudosiert. Durch gleichzeitiges Einleiten einer 15 Gew.-%igen Erdalkalilauge wird der pH-Wert konstant bei 2,2 gehalten. Nach etwa 1/4 h Unterbrechung, während dieser weiter gerührt wurde, stellte man den pH-Wert der Lösung durch Zudosieren von verdünnter Salzsäure auf 1,8 ein. Anschließend begann man mit der Zugabe von 5,6 L einer Lösung von 150 g $TiCl_4$ und 50 g HCl pro Liter. Durch gleichzeitiges Einleiten einer 15 Gew.-%igen Erdalkalilauge wurde der pH-Wert konstant gehalten.

Am Ende der Zugabe erhielt man ein tiefrotes Perlglanzpigment. Die Suspension wurde nach 1 h Nachrühren abgekühlt, über einen Büchnertrichter gesaugt und mit VE-Wasser nahezu ionenfrei gewaschen.

Zuletzt wurde das Perlglanzpigment 20 Minuten lang bei 800°C kalziniert.

**Beispiel 1c: Interferenz blau, 2. Ordnung**

100 g plättchenförmiger synthetischer Glimmer aus Beispiel 1$\alpha$ (Feststoffgehalt: 42,3 Gew.-%) wurden in 200 mL VE-Wasser suspendiert. Durch Zudosieren von verdünnter Salzsäure wurde ein pH-Wert von 2,2 eingestellt und die Suspension auf 80°C erhitzt. Dann wurden 50 mL einer Zinnchloridlösung mit c(Sn) = 24 g/L innerhalb 90 min zudosiert. Durch gleichzeitiges Einleiten einer 15 Gew.-%igen Erdalkalilauge wurde der pH-Wert konstant bei 2,2 gehalten. Nach etwa 1/4 h Unterbrechung, während dieser weiter gerührt wurde, stellte man den pH-Wert der Lösung durch Zudosieren von verdünnter Salzsäure auf 1,8 ein. Anschließend begann man mit der Zugabe von 7,2 L einer Lösung von 150 g $TiCl_4$ und 50 g HCl pro Liter. Durch gleichzeitiges Einleiten einer 15 Gew.-%igen Erdalkalilauge wurde der pH-Wert konstant gehalten.

Am Ende der Zugabe erhielt man einen tiefblaues Perlglanzpigment. Die Suspension wurde nach 1 h Nachrühren abgekühlt, über einen Büchnertrichter gesaugt und mit VE-Wasser nahezu ionenfrei gewaschen.

Zuletzt wurde das Perlglanzpigment 20 Minuten lang bei 800°C kalziniert.

**Vergleichsbeispiel 1: Interferenz silber**

Kommerziell erhältliches $TiO_2$-beschichtetes silbernes Perlglanzpigment SunShine Super White der Fa. Sun-Chemical.

**Beispiel 2: Bronze**

100 g plättchenförmiger synthetischer Glimmer aus Beispiel 1$\alpha$ (Feststoffgehalt: 42,3 Gew.-%) wurden in 200 mL VE-Wasser suspendiert. Durch Zudosieren von verdünnter Salzsäure wurde ein pH-Wert von 2,9 eingestellt und die Suspension auf 75°C erhitzt. Anschließend wurde mit einer Dosierrate von 150 mL/h eine Eisensulfatlösung zugegeben, die 65 g $Fe_2(SO_4)_3$ x 9 $H_2O$ und 1 mL konzentrierte Schwefelsäure je 100 mL Lösung enthielt. Durch gleichzeitige Zugabe einer 15 Gew.-%igen Erdalkalilauge wurde der pH-Wert durch Eindosierung auf 3,8 gehalten. Das Eisenhydroxid fiel als bräunlicher Niederschlag aus und lagert sich auf den Pigmentteilchen ab.

Nach Zugabe von 1500 mL $Fe_2(SO_4)_3$ - Lösung wurde die Belegung abgebrochen, 1 h bei Reaktionstemperatur nachgerührt, abgekühlt, über einen Büchnertrichter gesaugt und mit VE-Wasser nahezu ionenfrei gewaschen.

Das Perlglanzpigment wurde 20 Minuten bei 780°C kalziniert.

Das so erhaltene Perlglanzpigment besaß eine bronzefarbene Farbcharakterisitik mit gutem Glanz bei gleichzeitig hoher Streudichte.

Anwendungstechnische Beispiele

Beispiel **3:** Body **Lotion**

| INCI Name | Produktname | Gew.-% | Lieferant |
|---|---|---|---|
| **Phase A** | | | |
| Water | | 81.20 | |
| Carbomer | Acritamer 934 | 0.50 | www.ritacorp.com |
| Propylene Glycol | 1,2 Propandiol | 2.75 | www.vwr.com |
| Glycerin | Pricerine 9090 | 0.50 | www.uniqema.com |
| **Phase B** | | | |
| | **Gemäß Bsp. 1a (Silber)** | 2.00 | |
| Isopropyl Palmitate | Rita IPP NF | 2.00 | www.ritacorp.com |
| Glyceryl Stearate | Imwitor 960 K | 2.00 | www.sasolwax.com |
| Stearic Acid | Kortacid 1895 | 2.00 | www.akzonobel.com |
| Butyrospermum Parkii Butter (Shea Butter) | Shea Butter | 2.00 | www.jandekker.com |
| Cetyl Alcohol | Cetyl Alcohol | 1.00 | www.vwr.com |
| Cyclomethicone | Dow Corning 345 Fluid | 0.20 | www.dowcorning.com |
| Dimethicone | Dow Corning 200 Fluid/350 cst | 0.20 | www.dowcorning.com |
| Isostearyl Lactate | Patlac IL | 2.00 | www.ritacorp.com |
| Methylparaben, Propylparaben | Rokonsal SSH-1 | 0.30 | www.biochema.com |
| Tocopheryl Acetate | DL-Alpha-Tocopherolacetat | 0.10 | www.roche.com |
| **Phase C** | | | |
| Triethanolamine | Triethanolamine | 0.75 | www.vwr.com |
| Panthenol | D-Panthenol 75 L | 0.50 | www.basf.com |
| Fragrance | Palma Energy DF05 | q.s. | www.bell-europe.com |

Die Menge an eingesetztem Perlglanzpigment kann in einem Bereich von 0,1 Gew.-% bis 5,0 Gew.-% variiert werden. Diese Variation kann durch eine entsprechende Erhöhung oder Reduzierung der zugegebenen Wassermenge ausgeglichen werden. Phase A und Phase B wurden unter Rühren separat auf 80°C erhitzt. Anschließend wurde Phase B langsam unter Rühren zur Wasserphase hinzugefügt. Die Mischung wurde auf 50°C abgekühlt und Phase C hinzugefügt. Anschließend wurde solange weitergerührt bis Raumtemperatur erreicht war.

**Beispiel 4: Haar Mascara**

| INCI Name | Produktname | Gew.-% | Lieferant |
|---|---|---|---|
| **Phase A** | | | |
| Polyquaternium-16 | Luviquat FC 905 (Luviquat Exellence) | 2.70 | www.basf.com |
| Propylene Glycol | 1,2-Propanediol | 1.80 | www.vwr.com |
| Methylparaben | Methyl-4-hydroxybenzoate | 0.20 | www.sigmaaldrich.com |
| Water | | 64.45 | |

(fortgesetzt)

| Phase B | | | |
|---|---|---|---|
| Cetearyl Alcohol | Lanette O | 5.00 | www.cognis.com |
| Dimethicone | Dow Corning 200 Fluid/ 350 cst | 1.00 | www.dowcorning.com |
| Ceteareth-25 | Cremophor A 25 | 2.00 | www.basf.com |
| Propylparaben | Propyl-4-hydroxybenzoat | 0.10 | www.sigmaaldrich.com |
| **Phase C** | | | |
| Hydroxypropylcellulose | Klucel G | 0.50 | www.herc.com |
| Magnesium Aluminium Silicate | Veegum HV | 0.50 | www.rtvanderbilt.com |
| | Water | 19.00 | |
| **Phase D** | | | |
| | **Gemäß Bsp. 2 (Bronze)** | 2.50 | |
| Phenoxyethanol (and) Methylparaben (and) Butylparaben (and) Ethylparaben (and) Propylparaben (and) Isobutylparaben | Phenonip | 0.20 | www.clariant.com |
| Fragrance | Blue Shadow ÖKO | 0.05 | www.bell-europe.com |

Die Menge an eingesetztem Perlglanzpigment kann in einem Bereich von 0,5 Gew.-% bis 10,0 Gew.-% variiert werden. Diese Variation kann durch eine entsprechende Erhöhung oder Reduzierung der zugegebenen Wassermenge ausgeglichen werden. Phase A und Phase B wurden getrennt auf 80°C erhitzt, danach wurde Phase B langsam zu Phase A hinzugefügt. In einem separaten Gefäß wurden Klucel und Veegum dem Wasser von Phase C unter Rühren hinzugegeben. Anschließend wurde Phase AB auf 40°C abgekühlt und während des Abkühlens Phase C und D unter Rühren hinzugemischt.

**Beispiel 5: Lip Gloss**

| INCI Name | Produktname | Gew-% | Lieferant |
|---|---|---|---|
| **Phase A** | | | |
| Hydrogenated Polyisobutene (and) Ethylene/Propylene/Styrene Copolymer (and) Butylene/Ethylene/Styrene Copolymer | Versagel ME 750 | ad 100 | www.penreco.com |
| Simmondsia Chinensis (Jojoba) Seed Oil | Jojoba Oil - Natural/Golden | 2.00 | www.biochemica.com |
| Caprylyl Trimethicone | Silcare Silicone 31M50 | 7.00 | www.clariant.com |
| Stearyl Dimethicone | Silcare Silicone 41M65 | 3.20 | www.clariant.com |
| Hydrogenated Polydecene | Nexbase 2002 | 4.00 | www.jandekker.com |
| Isopropyl Myristate | Isopropyl Myristate | 4.50 | www.vwr.com |

(fortgesetzt)

| Phase B | | | |
|---|---|---|---|
| | **Gemäß Bsp. 1a (Silber)** | 5.00 | |
| Propylparaben | Propyl-4-hydroxybenzoat | 0.20 | www.sigmaaldrich.com |

Die Menge an eingesetztem Perlglanzpigment kann in einem Bereich von 0,1 Gew.-% bis 8,0 Gew.-% variiert werden. Diese Variation kann durch eine entsprechende Erhöhung oder Reduzierung der zugegebenen Menge an Versagel ME 750 ausgeglichen werden.

Phase A wurde auf 85°C erhitzt, anschließend wurden die Inhaltstoffe der Phase B einzeln zu Phase A hinzugegeben und gerührt bis eine gleichmäßige Konsistenz entstand und dann in ein Lip Gloss Gefäß abgefüllt.

**Beispiel 6: Gepresster Lidschatten**

| INCI Name | Produktname | Gew.-% | Lieferant |
|---|---|---|---|
| **Phase A** | | | |
| Mica | Silk Mica | 17.00 | www.vwr.com |
| Boron Nitride | Softouch CCS 102 | 2.50 | www.advceramicscos.com |
| Zinc Stearate | Kemilub EZ-V | 7.00 | www.undesa.com |
| Talc | **Talc Powder** | 38.50 | www.riedeldehaen.com |
| | **Gemäß Bsp. 1a (Silber)** | **25.00** | |
| **Phase B** | | | |
| Dimethicone | Dow Corning® 200 Fluid 5 cst | 5.00 | www.dowcorning.com |
| Cyclomethicone (and) Dimethicone Crosspolymer | Dow Corning® 9040 Elastomer | 5.00 | www.dowcorning.com |

Die Menge an eingesetztem Perlglanzpigment kann in einem Bereich von 5,0 Gew.-% bis 40,0 Gew.-% variiert werden. Diese Variation kann durch eine entsprechende Erhöhung oder Reduzierung der zugegebenen Menge an Mica ausgeglichen werden. Phase A wurde für 30 s bei 2500 rpm in einem Hochgeschwindigkeitsmixer gemischt. Anschließend wurde Phase B zugegeben und die Mischung für 60 s bei 3000 rpm im gleichen Mixer gemischt. Zuletzt wurde die Pulvermischung mittels einer Lidschattenpresse bei 150 bar für 30 s in Form gepresst.

**Beispiel 7: Lippenstift**

| INCI Name | Produktname | Gew-% | Lieferant |
|---|---|---|---|
| **Phase A** | | | |
| Carnauba Wax | Ewacera 34 | 4.50 | www.wagnerlanolin.de |
| Bees Wax | Ewacera 12 | 3.50 | www.wagnerlanolin.de |
| Candelilla Wax | Ewacera 42 | 4.00 | www.wagnerlanolin.de |

(fortgesetzt)

| Microcrystalline Wax | Parcera MW | 7.20 | www.paramelt.com |
|---|---|---|---|
| Cetyl Palmitate | Walrath synthetic | 2.00 | www.kahlwax.de |
| Hydrogenated Coco-Glycerides | Softisan 100 | 5.00 | www.sasolwax.com |
| Petrolatum | Penreco Blond | 5.80 | www.penreco.com |
| Cetearyl Octanoate | Luvitol EHO | 10.70 | www.basf.com |
| Tocopheryl Acetate | D,L-Alpha-Tocopherolacet at | 0.50 | www.dsm.com |
| Castor Oil | Castor Oil | 36.60 | www.riedeldehaen.com |
| *Phase B* | | | |
| Mica (and) Iron Oxide | Prestige Firered | 16.00 | www.eckart.net |
| | **Gemäß Bsp. 1a (Silber)** | 4.00 | |
| Methylparaben, Propylparaben | Rokonsal SSH-1 | 0.20 | www.biochema.com |

Die Menge an eingesetztem Perlglanzpigment kann in einem Bereich von 0,5 Gew.-% bis 10,0 Gew.-% variiert werden. Der Ausgleich kann mit anderen Pigmenten erfolgen, die Pigmentierung muss bei 21 Gew.-% gehalten werden.

Phase A wurde auf 85°C erhitzt, danach wurde Phase B der Phase A hinzugegeben und gemischt. Anschließend wurde die Mischung mit einer Temperatur von 75°C in einem Lippenstiftform abgefüllt.

## I Physikalische Charakterisierung

### Ia Teilchengrößenmessung

Die Pigmente der erfindungsgemäßen Beispiele und des Vergleichsbeispiels sowie der synthetische Glimmer aus Beispiel 1$\alpha$ wurden mittels Laserbeugungsmethoden charakterisiert (Gerät: Cilas 1064, Fa. Quantachrome). Die Auswertung der Streulichtsignale erfolgte nach der Fraunhofer Methode.

Hierzu wurden ca. 50 mL Perlglanzpigment- bzw. synthetischer Glimmersuspension (nicht flüchtiger Anteil ca. 35 Gew.-%) mit 50 mL Isopropanol über einem Magnetrührer vermischt und anschließend 300 Sekunden im Ultraschallbad Sonorex IK 52 der Fa. Bandelin behandelt. 2,5 mL Probensubstanz wurden dann zur Messung in das Gerät pipettiert.

Die $d_{50}$-Werte der volumengemittelten Summenhäufigkeitsverteilung sind in Tabelle 3 dargestellt (Spalte 8).

### Ib Bestimmung der mittleren Dicke des Substrates

Die mittlere Substratdicke wurde nach verschiedenen Methoden bestimmt. Die Ergebnisse sind in Tabelle 3 aufgeführt.

a) Die Perlglanzpigmente wurden 10 Gew.-%ig in einem 2K Klarlack Autoclear Plus HS der Fa. Sikkens GmbH mit einem Hülsenpinsel eingearbeitet und mit Hilfe einer Spiralrakel (26 $\mu$m Nassfilmdicke) auf eine Folie appliziert und getrocknet. Nach 24 h Abtrocknungszeit wurden von diesen Rakelabzügen Querschliffe angefertigt. Die Querschliffe wurden im REM vermessen. Hierbei wurden pro Probe mindestens 100 Pigmentteilchen vermessen, um eine aussagefähige Statistik zu erhalten. Dabei wurde neben der Substratschichtdicke auch die Schichtdicke der Metalloxidschicht bestimmt.

b) Die Perlglanzpigmente wurden gemäß der in der WO 2004/087816 A2 beschrieben Methode präpariert und ebenfalls im REM vermessen. Die Ergebnisse sind in Tabelle 3 in Spalte 10 dargestellt.

c) Die mittlere Substrathöhe wurde nach Gl, 7 berechnet. Hierbei wurde für den Substratradius die Hälfte der $d_{50}$-Werte der volumengemittelten Größenverteilung eingesetzt.

Die Gehalte an $TiO_2$ bzw. an $Fe_2O_3$ sowie an Substratmaterial wurden mittels RFA (Röntgenfluoreszenzanalyse) bestimmt.

Hierzu wurde das Perlglanzpigmentpulver direkt aus der Schüttung in ein Probenbehältnis, welches mit einer Polypropylenfolie 6 $\mu$m (Fa. Fluxana) überzogen wurde, gegeben und daraus vermessen. Als Messgerät diente das Gerät: Advant-X der Fa. Thermo ARL.

Die Metalloxidgehalte nach Gl. 1 sind in Tabelle 3 in Spalte 4 in Gew.-% bezogen auf Metalloxid und Substrat aufgeführt.

Schließlich musste noch die Schichtdicke von $TiO_2$ bzw. $Fe_2O_3$ bestimmt werden. Hier arbeitete man anhand der Farben der Pigmente und der in der Literatur hierzu veröffentlichten üblichen Schichtdicken. Diese Oxidschichtdicken sind in Tabelle 3 in Spalte 6 in nm aufgeführt.

Die nach Gl. 7 berechneten Werte sind in Tabelle 3 in Spalte 9 in nm aufgeführt.

**Tab 3: Physikalische Charakterisierung der Beispiele**

| Probe | Substrat | Metalloxid | Gehalt Metalloxid (Gl. 1) in % | Farbe | Schichtdicke Metalloxid / nm | | $d_{50}$ [μm] (Gerät: Cilas 1064) | hs [nm] | $h_s$ aus REM am Glimmer [nm] | | $h_s$ aus REM am Perlglanz im Schliff [nm] |
|---|---|---|---|---|---|---|---|---|---|---|---|
| | | | | | aus Farbe | aus REM | | nach Gl. 7 | Pulver | Schliff | |
| Beispiel 1a | Glimmer n. Bsp. 1α | $TiO_2$ | 64.0 | silber | 50 | 53 | 3,6 | 95 | | | 114 |
| Beispiel 1b | Glimmer n. Bsp. 1α | $TiO_2$ | 79.1 | rot | 90 | 89 | 3,8 | 81 | 79 | 90 | 111 |
| Beispiel 1c | Glimmer n. Bsp. 1α | $TiO_2$ | 82.9 | blau | 130 | 131 | 3,9 | 96 | | | |
| Beispiel 2 | Glimmer n. Bsp. 1α | $Fe_2O_3$ | 66.1 | bronze | 40 | 42 | 3,7 | 82 | 79 | 90 | 112 |

**Ergebnisse der Schichtdickenbestimmungen**

Es wird eine gute Übereinstimmungen der über die Oxidgehalte und -schichtdicken nach Gl. 7 berechneten Werte mit den mittleren Schichtdicken, die nach der Auswertung der senkrecht orientierten Pulver des Glimmers (Spalte 10) bzw. des Glimmers im Rakelabzug gefunden wurden, festgestellt.

Diese Befunde sprechen für die Konsistenz des Modells der Gl. 1 - 7 und die Zuverlässigkeit der Ermittlung der mittleren Schichtdicke nach dieser Methode.

Die Bestimmung der mittleren Schichtdicke der Glimmer anhand der Querschliffe (Spalte 11) weist systematisch höhere Werte im Vergleich zur Bestimmung an senkrecht orientierten Pulvern auf. Dies dürfte im Wesentlichen darauf zurückzuführen sein, dass die Pigmente der Querschliffmethode etwas unterschiedliche Orientierungen der Plättchen innerhalb des Lacks aufweisen.

Die Bestimmung der mittleren Schichtdicke $h_s$ anhand der Querschliffe der Perlglanzpigmente (Spalte 12) selbst führt zu potentiell noch höheren Werten.

Daher wird im Rahmen dieser Erfindung die mittlere Substratschichtdicke bevorzugt nach Gl. 7 ermittelt, wenn die geometrischen Schichtdicken der optisch aktiven Schicht 40 nm bis 180 nm betragen.

**II Abprüfungen:**

**IIa Winkelabhängige Helligkeitsmessungen**

Zur Charakterisierung des reflektiven Streulichtanteiles wurde das Perlglanzpigment mit einer Pigmentierungshöhe von 6 Gew.-% bezogen auf das Gesamtgewicht des Nasslacks in einen konventionellen Nitrocelluloselack (Dr. Renger Erco Bronzemischlack 2615e; Fa. Morton) eingerührt. Dabei wurde das Perlglanzpigment vorgelegt und anschließend mit einem Pinsel in den Lack dispergiert. Der fertige Lack wurde auf einem handelsüblichen maschinellen Rakelabzugsgerät (Spiralrakel) mit einer Nassfilmdicke von 50 $\mu$m auf Prüfkarten Nr. 2853 der Fa. Byk Gardner (Kontrastpapier) appliziert.

Mit dem Mehrwinkelfarbmessgerät MA 68II der Firma X-Rite wurde bei konstantem Einfallswinkel von 45° (gemäß Herstellerangaben) und einem Beobachtungswinkel von 110° relativ zum Glanzwinkel der L*-Wert bestimmt.

Stark reflektierende Proben (Idealfall Spiegel) reflektieren nahezu das gesamte eintreffende Licht im sog. Glanzwinkel. Je weiter man sich bei der Messung also vom Glanzwinkel entfernt, desto weniger Licht und somit Helligkeit (L*) kann gemessen werden. In der Lackindustrie wird dieser Effekt, der bevorzugt bei Metallpigmenten auftritt, als sog. Helligkeitsflop beschrieben.

Anders ist es bei stark streuenden Proben. Hier wird das einfallende Licht im Idealfall gleichmäßig über alle Winkel reflektiert. Somit sollten auch bei Messwinkeln weit entfernt vom Glanzwinkel noch beträchtliche Helligkeitswerte detektierbar sein. Hier ist zur Charakterisierung vor allem der 110°-Winkel geeignet.

**IIb Glanzmessungen:**

Der streuende Charakter der jeweiligen Probe lässt sich zusätzlich durch die Messung des Glanzes charakterisieren. Der Glanz ist ein Maß für die gerichtete Reflexion. Stark streuende Proben sollten mithin einen niedrigen Glanz aufweisen. Es wurden die Nitrolackapplikationen aus IIa mit Hilfe eines Micro-Tri-Gloss Glanzmessgerätes der Fa. Byk Gardner mit einem Messwinkel von 60° auf schwarzem Hintergrund vermessen.

**IIc Deckung**

Die erfindungsgemäßen Perlglanzpigmente und das Vergleichsbeispiel wurden in verschiedenen Konzentrationen auf Prüfkarten Nr. 2853 der Fa. Byk Gardner (Kontrastpapier) gemäß II a appliziert und die Deckfähigkeit visuell verglichen (Tabelle 4). Dabei wurde die Deckfähigkeit anhand folgender Noten beurteilt:

1 = sehr schlecht
2 = schlecht
3 = mittel
4 = gut
5 = sehr gut

**IId Bestimmung des Bleigehalts**

Der Bleigehalt wurde über Feststoff-Graphitrohr-Atomabsorptionsspektrometrie bestimmt. Als Gerät wurde ein ZEENIT 650 mit Feststoffprobengeber SSA 600 (Hersteller: Analytik Jena) eingesetzt. Die Bleigehalte der erfindungsgemäßen Perlglanzpigmente sind Tabelle 4 zu entnehmen.

Tabelle 4: Ergebnisse der optischen Messungen, Deckfähigkeit und Bleigehalt

| Interferenzfarbe | Probe | Deckung (visuell) | L* 110° | Glanz 60° | Bleigehalt [ppm] |
|---|---|---|---|---|---|
| silber | Beispiel 1 a | 4 | 38,9 | 9,9 | < 1 |
| rot | Beispiel 1 b | 4 | | | < 1 |
| blau | Beispiel 1 c | 4 | 37,5 | 6,4 | < 1 |
| bronze | Beispiel 2 | 5 | 22,7 | 11,5 | < 1 |
| silber | Vergleichsbeispiel 1 | 2 | 13,1 | 55,8 | 16 |

Die erfindungsgemäßen Perlglanzpigmente weisen eine durchgehend bessere Deckfähigkeit als das Vergleichsbeispiel aus dem Stand der Technik auf.

Weiterhin zeigen die Perlglanzpigmente der erfindungsgemäßen Beispiele einen geringeren Glanz als das Vergleichsbeispiel des korrespondierenden Farbtons. Die gerichtete Reflexion ist hier offenbar geringer. Dies ist für einen Softfokuseffekt durchaus erwünscht.

In gleicher Weise sind die $L_{110°}$-Werte bei den erfindungsgemäßen Beispielen wesentlich höher als bei dem korrespondierenden Vergleichsbeispiel. Die Messungen zeigen, dass das die erfindungsgemäßen Beispiele offenbar einen höheren Streulichtanteil aufweisen.

Dies ist vermutlich auf die geringe Partikelgröße und die geringe Substratschichtdicke zurückzuführen. Die geringe Partikelgröße des synthetischen Glimmers geht einher mit einem erhöhten Kantenanteil und damit mit mehr Streuung. Die geringere Substratschichtdicke wiederum führt zu einem wesentlich erhöhten $TiO_2$-Gehalt bei vergleichbarer Schichtdicke. Das hochbrechende $TiO_2$ fällt gewöhnlich nicht in einer perfekten glatten Schicht, sondern weist immer eine gewisse Korngrößenverteilung auf. Diese Oxidkörner verursachen immer eine gewisse Streuung. Es wird vermutet, dass der höheren Streulichtanteil der erfindungsgemäßen Proben auch auf diesen Effekt zurückzuführen ist.

Weiterhin weisen die erfindungsgemäßen Perlglanzpigmente einen signifikant niedrigeren Bleigehalt als das Vergleichsbeispiel 1 auf.

**IIe Messung des Softfokuseffektes**

Hierzu wurden die Perlglanzpigmente mit einer Pigmentierungshöhe von 2,5 Gew.-% (bezogen auf das Gesamtgewichtes des Naßlacks) in einen konventionellen Nitrocelluloselack (Dr. Renger Erco Bronzemischlack 2615e; Fa. Morton) eingerührt. Dabei wurde das Perlglanzpigment vorgelegt und anschließend mit einem Pinsel in den Lack dispergiert.

Der fertige Lack wurde auf einem handelsüblichen maschinellen Rakelabzugsgerät (Spiralrakel) mit einer Nassfilmdicke von 50 µm auf kommerziell erhältliche transparente PET-Folien, z.B. Hostophan, appliziert.

Die so beschichteten Folien wurden mit dem Haze-gard plus der Fa. Byk Gardner, auf Gesamttransmission und Trübung (Haze) vermessen. Die Trübung ergibt sich aus der sog. Großwinkelstreuung (lt. ASTM D 1003 ist der Haze die Lichtmenge, die im Mittel mehr als 2,5° vom einfallenden Lichtstrahl abweicht - gemessen in %).

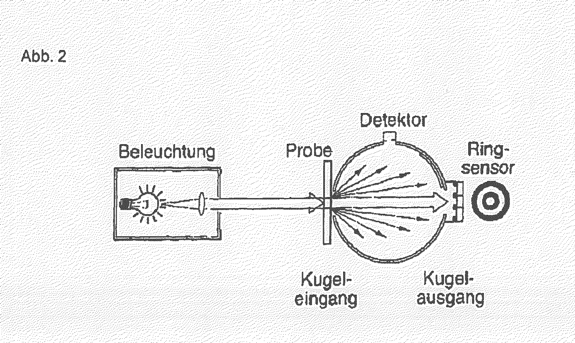

Das Messprinzip kann Abbildung 2 entnommen werden.

Ein Lichtbündel trifft auf die Probe und tritt in eine integrierende Kugel ein. Die Kugelinnenwand ist matt-weiß beschichtet, um eine gleichmäßige Verteilung des Lichtes zu garantieren. Ein Detektor in der Kugel misst die Gesamttransmission bei geschlossenem Kugelausgang und Haze bei geöffnetem Kugelausgang.

Um die gerichtete Reflexion zu messen, wurde der Glanz der jeweiligen Folien mit einem Micro-Gloss Gerät der Fa. Byk Gardener bei einem Winkel von 60°C bestimmt.

Tabelle 5: Messung des Softfokuseffektes

| Probe | Transmission | Haze (% bei > 2,5°) | Glanz |
|---|---|---|---|
| Beispiel 1a | 73,4 | 72,6 | 29,5 |
| Beispiel 1b | 85,6 | 71,5 | 33,7 |

Die Messwerte aus Tabelle 5 zeigen, dass die erfindungsgemäßen Perlglanzpigmente einen hohen Transmissionsgrad besitzen. Zugleich weisen diese Perlglanzpigmente aber auch einen sehr hohen Streulichtanteil auf (Haze).

In der Kombination der Eigenschaften erfüllen die erfindungsgemäßen Perlglanzpigmente wesentliche Anforderungen, um in der Applikation einen Softfokuseffekt zu erzielen.

Zusätzlich verbinden die beanspruchten Perlglanzpigmente im Gegensatz zu handelsüblichen Softfokuspartikeln die Eigenschaften eines Softfokuspigments mit denen eines Perlganzpigments (Interferenzfarbtöne, seidiger Glanz) bei gleichzeitig geringem Bleigehalt.

**Patentansprüche**

1. Perlglanzpigmente umfassend ein weitgehend transparentes plättchenförmiges synthetisches Substrat mit einer Dichte $\rho_s$ und mindestens einer optisch wirksamen Beschichtung mit einer Dichte $\rho_M$, **dadurch gekennzeichnet,**

   **dass** das Substrat eine mittlere Größe $d_{50}$ aus einem Bereich von 2,0 $\mu$m bis 8,0 $\mu$m, eine mittlere Höhe $h_s$ aus einem Bereich von 40 nm bis 110 nm aufweist und der Gesamtbleigehalt der Perlglanzpigmente bei $\leq$ 10 ppm liegt,
   wobei die optisch wirksame Beschichtung eine hochbrechende Beschichtung mit einem Brechungsindex $n_M > 2,0$ ist und
   die optisch wirksame Schicht eine Metalloxidschicht ist, wobei a) die Metalloxidschicht aus $TiO_2$ und das Substrat aus synthetischem Glimmer besteht und folgender Zusammenhang zwischen dem TiOz-Gehalt in Gew.-%, bezogen auf das Gesamtgewicht von $TiO_2$ und synthetischem Glimmer, und der mittleren Schichtdicke der $TiO_2$-Beschichtung besteht:

   ein Metalloxidgehalt von 30 - 80 Gew.-% bei einer mittleren Metalloxidschichtdicke von über 20 bis 50 nm;
   ein Metalloxidgehalt von 50 - 85 Gew.-% bei einer mittleren Metalloxidschichtdicke von über 50 bis 75 nm:
   ein Metalloxidgehalt von 59 - 89 Gew.-% bei einer mittleren Metalloxidschichtdicke von über 75 bis 95 nm;
   ein Metalloxidgehalt von 66 - 92 Gew.-% bei einer mittleren Metalloxidschichtdicke von über 95 bis 125 nm;
   ein Metalloxidgehalt von 69 - 96 Gew.-% bei einer mittleren Metalloxidschichtdicke von über 125 bis 215 nm; oder
   wobei b) die Metalloxidschicht aus $Fe_2O_3$ und das Substrat aus synthetischem Glimmer besteht und folgender Zusammenhang zwischen dem $Fe_2O_3$-Gehalt in Gew.-%, bezogen auf das Gesamtgewicht von $Fe_2O_3$ und synthetischem Glimmer, und der mittleren Schichtdicke der $Fe_2O_3$-Beschichtung:

   ein $Fe_2O_3$-Gehalt von 47,5 - 72,4 Gew.-% bei einer mittleren $Fe_2O_3$-Schichtdicke von über 35 bis 45 nm;
   ein $Fe_2O_3$-Gehalt von 57,5 - 82,4 Gew.-% bei einer mittleren $Fe_2O_3$-Schichtdicke von über 45 bis 55 nm;
   ein $Fe_2O_3$-Gehalt von 62,5 - 87,4 Gew.-% bei einer mittleren $Fe_2O_3$-Schichtdicke von über 55 bis 65 nm.

2. Perlglanzpigmente nach Anspruch 1,
   **dadurch gekennzeichnet,**
   **dass** die Perlglanzpigmente eine Größenverteilung mit einem $d_{90}$-Wert aus einem Bereich von 5,0 $\mu$m bis 11,0 $\mu$m aufweisen.

3. Perlglanzpigmente nach einem der vorstehenden Ansprüche,
   **dadurch gekennzeichnet,**

   **dass** die mittlere Substrathöhe $h_s$ durch folgende Formel bestimmt wird;

$$h_S = \frac{\frac{4d_M^3}{3r_s^2} + \frac{\pi d_M^2}{r_s} + 2 \cdot d_M}{\frac{\rho_S}{\rho_M \cdot (\frac{100}{c_M} - 1)} - \left(\frac{d_M}{r_s}\right)^2 - 2 \cdot \frac{d_M}{r_s}},$$

   unter der Voraussetzung, dass die mittlere geometrische Schichtdicke der optisch wirksamen Schicht $d_M$ 40 nm bis 180 nm ist,

$r_s = d_{50}/2$ der mittlere Radius des Substrates bestimmt über Laserbeugungsmethoden, $\rho_s$ die Dichte des Substrates und $\rho_M$ die Dichte der optisch wirksamen Beschichtung und $c_M$ der Gewichtsanteil in % der optisch wirksamen Beschichtung bestimmt über analytische Messungen, bezogen auf das Gesamtgewicht von Substrat und optisch wirksamer Beschichtung ist.

4. Verfahren zur Herstellung der Perlglanzpigmente nach einem der Ansprüche 1 bis 37,
**dadurch gekennzeichnet,**
**dass** es folgende Schritte umfasst:

    a) Klassieren des weitgehend transparenten plättchenförmigen synthetischen Substrates unter Erhalt eines Substrates mit einer mittleren Höhe $h_s$ aus einem Bereich von 40 nm bis 110 nm.
    b) Beschichten des klassierten weitgehend transparenten plättchenförmigen synthetischen Substrates mit mindestens einer optisch wirksamen, vorzugsweise hochbrechenden, Schicht unter Erhalt eines Perlglanzpigmentes mit einer mittleren Größe $d_{50}$ aus einem Bereich von 2,1 $\mu$m bis 8,6 $\mu$m.

5. Verwendung eines der Perlglanzpigmente nach einem der Ansprüche 1 bis 3 in Lacken, Druckfarben, Tintenstrahltinten, Tonern, Kosmetika, Kunststoffen, Textilien, Glas, Email, Glasuren oder Keramik.

6. Beschichtungsmittel, insbesondere kosmetisches Präparat, enthaltend eines der Perlglanzpigmente nach einem der Ansprüche 1 bis 37.

**Claims**

1. Pearlescent pigments comprising a largely transparent, platelet-shaped synthetic substrate having a density $\rho_s$ and at least one optically active coating with a density $\rho_M$,
**characterised in that**

    the substrate has an average size $d_{50}$ in a range of from 2.0 $\mu$m to 8.0 $\mu$m, an average height $h_s$ in a range of from 40 nm to 110 nm, and the total lead content of the pearlescent pigments is $\leq$ 10 ppm, wherein the optically active coating is a highly refractive coating with a refractive index $n_M > 2.0$ and
the optically active layer is a metal oxide layer,
wherein a) the metal oxide layer comprises $TiO_2$ and the substrate comprises synthetic mica and the correlation between the $TiO_2$ content as a % by weight, based on the total weight of $TiO_2$ and synthetic mica, and the average layer thickness of the $TiO_2$ coating is as follows:

    a metal oxide content of 30 to 80 % by weight with an average metal oxide layer thickness of over 20 to 50 nm;
    a metal oxide content of 50 to 85 % by weight with an average metal oxide layer thickness of over 50 to 75 nm;
    a metal oxide content of 59 to 89 % by weight with an average metal oxide layer thickness of over 75 to 95 nm;
    a metal oxide content of 66 to 92 % by weight with an average metal oxide layer thickness of over 95 to 125 nm;
    a metal oxide content of 69 to 96 % by weight with an average metal oxide layer thickness of over 125 to 215 nm;
    or
    wherein b) the metal oxide layer comprises $Fe_2O_3$ and the substrate comprises synthetic mica and the correlation between the $Fe_2O_3$ content as a % by weight, based on the total weight of $Fe_2O_3$ and synthetic mica, and the average layer thickness of the $Fe_2O_3$ coating is as follows:

    a $Fe_2O_3$ content of 47.5 to 72.4 % by weight with an average $Fe_2O_3$ layer thickness of over 35 to 45 nm;
    a $Fe_2O_3$ content of 57.5 to 82.4 % by weight with an average $Fe_2O_3$ coating thickness of over 45 to 55 nm;
    a $Fe_2O_3$ content of 62.5 to 87.4 % by weight with an average $Fe_2O_3$ content of over 55 to 65 nm.

2. Pearlescent pigments as claimed in claim 1,
**characterised in that**
the pearlescent pigments have a size distribution with a $d_{90}$ value in a range of from 5.0 $\mu$m to 11.0 $\mu$m.

3. Pearlescent pigments as claimed in one of the preceding claims,
**characterised in that**

the average substrate height $h_s$ is determined by means of the following formula:

$$h_s = \frac{\dfrac{4d_M^3}{3r_s^2} + \dfrac{\pi d_M^2}{r_s} + 2.d_M}{\dfrac{\rho_s}{\rho_M.\left(\dfrac{100}{c_M} - 1\right)} - \left(\dfrac{d_M}{r_s}\right)^2 - 2.\dfrac{d_M}{r_s}}$$

on the proviso that the average geometric layer thickness of the optically active layer $d_M$ is 40 nm to 180 nm, $r_s = d_{50}/2$ is the average radius of the substrate, determined by laser refraction methods, $\rho_s$ is the density of the substrate and $\rho_M$ the density of the optically active coating and $c_M$ is the proportion by weight as a % of the optically active coating, determined by analytical measurements, based on the total weight of substrate and optically active coating.

4. Method of producing pearlescent pigments as claimed in one of claims 1 to 3,
**characterised in that**
it comprises the following steps:

a) classifying the largely transparent, platelet-shaped synthetic substrate to obtain a substrate having an average height $h_s$ within a range of from 40 nm to 110 nm,
b) coating the classified largely transparent platelet-shaped synthetic substrate with at least one optically active, preferably highly refractive layer to obtain a pearlescent pigment with an average size $d_{50}$ in a range of from 2.1 $\mu$m to 8.6 $\mu$m.

5. Use of one of the pearlescent pigments as claimed in one of claims 1 to 3 in paints, printing inks, inkjet inks, toners, cosmetics, plastics, textiles, glass, enamel, glazes or ceramics.

6. Coating composition, in particular a cosmetic preparation, containing one of the pearlescent pigments as claimed in one of claims 1 to 3.

**Revendications**

1. Pigments nacrés comprenant un substrat synthétique lamellaire essentiellement transparent, ayant une masse volumique $\rho_S$ et au moins un revêtement optiquement actif ayant une masse volumique $\rho_M$, **caractérisés en ce que**

le substrat présente une granulométrie moyenne $d_{50}$ comprise dans la plage de 2,0 $\mu$m à 8,0 $\mu$m, une hauteur moyenne $h_s$ comprise dans la plage de 40 nm à 110 nm, la teneur totale en plomb des pigments nacrés étant $\leq$ 10 ppm,
dans lequel le revêtement optiquement actif est un revêtement à grand indice de réfraction, ayant de préférence un indice de réfraction $n_M > 2,0$.
dans lequel la couche optiquement active comprend ou est une couche d'oxyde métallique,
dans lequel a) la couche d'oxyde métallique étant constituée de $TiO_2$, et le substrat est constitué de mica synthétique, et la relation suivante existe entre la teneur en $TiO_2$ en % en poids, par rapport au poids total du $TiO_2$ et du mica synthétique, et l'épaisseur de couche moyenne du revêtement $TiO_2$,
une teneur en oxyde métallique de 30 à 80 % en poids pour une épaisseur moyenne de la couche d'oxyde métallique supérieure à 20 et allant jusqu'à 50 nm; une teneur en oxyde métallique de 50 à 85 % en poids pour une épaisseur moyenne de la couche d'oxyde métallique supérieure à 50 et allant jusqu'à 75 nm; une teneur en oxyde métallique de 59 à 89 % en poids pour une épaisseur moyenne de la couche d'oxyde métallique supérieure à 75 et allant jusqu'à 95 nm; une teneur en oxyde métallique de 66 à 92 % en poids pour une épaisseur moyenne de la couche d'oxyde métallique supérieure à 95 et allant jusqu'à 125 nm;
une teneur en oxyde métallique de 69 à 96 % en poids pour une épaisseur moyenne de la couche d'oxyde métallique supérieure à 125 et allant jusqu'à 215 nm, ou
b) la couche d'oxyde métallique est du $Fe_2O_3$, le substrat étant du mica synthétique, la relation suivante existant entre la teneur en $Fe_2O_3$ en pourcentage en poids, rapporté au poids total de $Fe_2O_3$ et de mica synthétique et à l'épaisseur de couche moyenne du revêtement de $Fe_2O_3$ ;

une teneur en $Fe_2O_3$ de 47,5 à 72,4 % en poids pour une épaisseur moyenne de couche de $Fe_2O_3$ supérieure à 35 et allant jusqu'à 45 nm ;

une teneur en $Fe_2O_3$ de 57,5 à 82,4 % en poids pour une épaisseur moyenne de couche de $Fe_2O_3$ supérieure à 45 et allant jusqu'à 55 nm ;

une teneur en $Fe_2O_3$ de 62,5 à 87,4 % en poids pour une épaisseur moyenne de couche de $Fe_2O_3$ supérieure à 55 et allant jusqu'à 65 nm.

2. Pigments nacrés selon la revendication 1, **caractérisés en ce que** les pigments nacrés présentent une distribution granulométrique ayant un $d_{90}$ compris dans la plage de 5,0 $\mu$m à 11,0 $\mu$m.

3. Pigments nacrés selon l'une des revendications précédentes, **caractérisés en ce que** la hauteur moyenne hs du substrat est déterminée par la formule suivante :

$$h_S = \frac{\dfrac{4d_M^3}{3r_S^2} + \dfrac{\pi d_M^2}{r_S} + 2 \cdot d_M}{\dfrac{\rho_S}{\rho_M \cdot (\dfrac{100}{c_M} - 1)} - \left(\dfrac{d_M}{r_S}\right)^2 - 2 \cdot \dfrac{d_M}{r_S}},$$

à la condition que l'épaisseur de couche géométrique moyenne de la couche optiquement active $d_M$ soit de 40 nm à 180 nm,

que $r_s = d_{50}/2$, le diamètre moyen du substrat, soit déterminé par des méthodes de diffraction laser,

que ps, la masse volumique du substrat, et $\rho_M$, la masse volumique du revêtement optiquement actif, et $c_M$, la proportion en poids en pourcentage du revêtement optiquement actif, soient déterminées par des mesures analytiques, par rapport au poids total du substrat et du revêtement optiquement actif.

4. Procédé de fabrication des pigments nacrés selon l'une des revendications 1 à 3, **caractérisé en ce qu'**il comprend les étapes suivantes :

a) classification du substrat synthétique lamellaire sensiblement transparent, avec obtention d'un substrat ayant une hauteur moyenne $h_s$ comprise dans la plage de 40 nm à 110 nm,

b) application sur le substrat synthétique lamellaire sensiblement transparent classifié d'au moins une couche optiquement active, ayant de préférence un grand indice de réfraction, avec obtention d'un pigment nacré ayant une granulométrie moyenne $d_{50}$ comprise dans la plage de 2,1 $\mu$m à 8,6 $\mu$m.

5. Utilisation de l'un des pigments nacrés selon l'une des revendications 1 à 3 dans les vernis, les encres d'imprimerie, les encres pour impression au jet d'encre, les toners, les produits cosmétiques, les matières plastiques, les textiles, le verre, l'émail, les glaçures ou la céramique.

6. Composition de revêtement, en particulier préparation cosmétique, contenant l'un des pigments nacrés selon l'une des revendications 1 à 3.

$$V_1 = 2 \pi r_S^2 d_M$$

$$V_2 = \tfrac{4}{3} \pi d_M^3 + \pi^2 d_M^2 r_S$$

$$V_3 = \pi d_M^2 h_S + 2\pi r_S d_M h_S$$

Substrat

Beschichtung

**Abb. 1: Substrat S und Beschichtung M**

Abb. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0723997 B1
- EP 1564261 A2
- EP 1072651 A1
- WO 2007054379 A1
- WO 02090448 A2
- WO 2009127406 A1
- EP 1727864 A1
- EP 1682622 A1
- WO 2004087816 A2

**In der Beschreibung aufgeführte Nicht-Patentliteratur**

- **NANCY M. HEPP ; WILLIAM R. MINDAK ; JOHN CHENG.** *J. Cosmet. Sci.,* Juli 2009, vol. 60, 405-414
- **HEPP N. M. et al.** *J. Cosmet. Sci.,* Juli 2009, vol. 60, 405-414
- **C. SCHMIDT ; M. FRITZ.** Optical Physics of Synthetic Interference Pigments. *Kontakte (Darmstadt),* 1992, (2), 15-24
- **F. HOFMEISTER.** *Farbe + Lack,* 1989, vol. 95, 557